# EUROPÄISCHE PATENTSCHRIFT

(11) **EP 4 104 900 B1**
(45) Veröffentlichungstag und Bekanntmachung des Hinweises auf die Patenterteilung: **19.11.2025**
(21) Anmeldenummer: 22178932.4
(22) Anmeldetag: 14.06.2022
(51) Int. Cl.: A61N 5/06, A61N 5/067, A61B 5/00, A61B 18/22, F21V 8/00

(54) **BELEUCHTUNGSSYSTEM MIT EINEM LICHTLEITER MIT EINEM DIFFUSOR-ELEMENT**
LIGHTING SYSTEM WITH A LIGHT GUIDE WITH A DIFFUSER ELEMENT
SYSTÈME D'ÉCLAIRAGE DOTÉ D'UN GUIDE DE LUMIÈRE DOTÉ D'UN ÉLÉMENT DIFFUSEUR

(30) Priorität: 15.06.2021 DE 102021115485
(43) Veröffentlichungstag der Anmeldung: 21.12.2022
(73) Patentinhaber: SCHOTT AG, 55122 Mainz (DE)
(72) Erfinder: SCHULTHEIS, Bernd, 55270 Schwabenheim (DE); HENN, Christian, 55546 Frei-Laubersheim (DE); KLINK, Tobias, 65428 Rüsselsheim (DE); KEIPER, Oliver, 65510 Hünstetten (DE); RUSSERT, Hubertus, 55270 Jugenheim (DE)
(74) Vertreter: Blumbach · Zinngrebe Patentanwälte PartG mbB

(56) Entgegenhaltungen:
- EP-A2- 0 761 257
- WO-A1-2017/174549
- DE-A1- 102017 122 756
- US-A- 4 466 697
- US-A1- 2009 275 931
- US-A1- 2019 159 867
- US-A1- 2020 289 202
- US-A1- 2020 405 391
- US-B1- 10 631 930

## Beschreibung

Die Erfindung betrifft ein Beleuchtungssystem, welches wenigstens eine Lichtquelle, einen Lichtleiter und ein optisches Element, welches bevorzugt als Diffusor-Element ausgebildet ist und an einem distalen Ende des Lichtleiters angeordnet ist, so dass Licht aus dem Lichtleiter in das optische Element einkoppelbar ist, aufweist.

Derartige Beleuchtungssysteme kommen verstärkt im medizinischen Umfeld zum Einsatz. Derzeit lassen sich folgende Anwendungsschwerpunkte klassifizieren:
- Photodynamische Therapie (PDT) bzw. Photo-Immuno-Therapie (PIT) zur Tumortherapie,
- Endovenöse Lasertherapie (EVLT) zur Behandlung von Krampfadern,
- Laser induzierte interstitielle Thermotherapie (LITT) sowie
- sonstige Anwendungen, u.a. im Bereich der Dentalmedizin, Augenheilkunde sowie Dermatologie, oder auch zur Behandlung von Epilepsie.

Die Photodynamische Therapie (PDT) ist eine minimal-invasive Therapiemöglichkeit bei verschiedenen Krebserkrankungen. Unter der PDT versteht man ein Verfahren zur Behandlung von Tumoren und anderen Gewebeveränderungen (wie beispielsweise Gefäßneubildungen) mit Licht in Kombination mit einer lichtaktivierbaren Substanz. Zu Beginn der Behandlung werden den Patienten intravenös lichtsensible Substanzen, sogenannte Photosensitizer in die Blutbahn injiziert, die sich in beziehungsweise an den Krebszellen anreichern. Diese natürlichen Photosubstanzen konzentrieren sich in den Tumorzellen und bewirken dort eine starke Lichtempfindlichkeit. Dazu werden während der PDT-Behandlung in das Tumor-Gewebe mehrere Kanülen (typischerweise bis zu 8) gestochen, in die jeweils eine Lichtquelle möglichst räumlich über das Tumor-Gewebe verteilt angeordnet sein müssen. Laser-Licht, in der Regel mit Wellenlängen im sichtbaren Spektralbereich, zum Beispiel Grün-Licht mit 532 nm oder Rot-Licht mit 690 nm Wellenlänge, leuchtet das Tumor-Gewebe möglichst gleichmäßig von innen aus. Dabei bilden sich in diesen Zellen aggressive Sauerstoffradikale, welche die Tumorzellen selektiv zerstören. Im Gegensatz zu den kranken Zellen sollen gesunde Zellen von dieser chemischen Reaktion unberührt bleiben. Der genaue Wirkmechanismus ist u.a. in "Photodynamic Therapy of Cancer", Cancer Medicine, 2003 beschrieben. Bei der Photo-Immuno-Therapie (PIT) wird mit entsprechend modifiziertem Photosensitizer dagegen eine Immun-Reaktion an bzw. in der Krebszelle ausgelöst, die bei Lichtbestrahlung zum Absterben der Krebszelle führt.

Bei den Lichtquellen wird üblicherweise zwischen Zylinder-Diffusoren und Spot-Diffusoren unterschieden, die einen vorwärts gerichteten Beleuchtungskegel erzeugen sowie Punktstrahler, die eine radiale Lichtemission aufweisen. Bei den Zylinder-Diffusoren kommt es im Betriebszustand insbesondere auf eine möglichst homogene seitliche Abstrahlung über deren Länge an. Sowohl axial, also an allen Punkten entlang jeder Linie vom proximalen zum distalen Ende in Richtung der Längsachse ist die Abstrahlungsintensität im Rahmen der Homogenitätsanforderung gleich, als auch radial, also an allen Punkten jeder Umfangslinie entlang der Längsachse ist die Abstrahlungsintensität im Rahmen der Homogenitätsanforderung annähernd gleich, womit die Zylinder-Diffusoren nahezu als Lambertsche Strahler wirken.

Gleichzeitig ist es auch vorteilhaft, wenn eine hohe Streueffizienz erzielt werden kann, um einen möglichst geringen Wärmeeintrag ins Gewebe sicher zu stellen. Dabei soll eine vorwärts gerichtete Abstrahlung, insbesondere aus dem distalen Ende heraus vermieden werden. Die typische Laser-Leistung liegt bei den PDT-Anwendungen bei < 5 W Dauerleistung, so dass pro cm Diffusorlänge maximal zwischen 100 mW und 1000 mW, typischerweise zwischen 200 mW und 500 mW, abgestrahlt werden.

Bestehende Beispiele sind Diffusor-Elemente aus einem dünnen Silikon-Zylinder, in die Streupartikel eingelagert sind. Die Schrift DE 10129029 A1 beschreibt eine flexible Vorrichtung zur thermischen Verödung von biologischem Gewebe mittels Laserstrahlung mit einem die Laserstrahlung führenden Lichtleiter, dessen distales Ende von einem für die Laserstrahlung transparenten oder opaken Hüllschlauch umgeben ist, welcher das Faserende überragt. Diese können allerdings nur sehr aufwendig kostenintensiv mit ausreichender Abstrahlhomogenität hergestellt werden, da Konglomerate der Streupartikel oft Abstrahlspots ergeben, bei denen die Intensität dann deutlich über dem Durchschnitt liegt.

Lichtleiter mit den Diffusor-Elementen werden in einigen Anwendungen nur einmal verwendet und nach jeder Behandlung entsorgt. Daher besteht auch ein gewisser Kostendruck, was die Herstellkosten betrifft. Daher wird verstärkt auch über wiederverwendbare Lösungen nachgedacht. Derartige Lösungen müssen dann entsprechend den einschlägig bekannten Normen aufbereitbar, beispielsweise desinfizierbar und/oder sterilisierbar sein. Als Aufbereitungsverfahren sind hier insbesondere Reinigungs-/Desinfektionsverfahren mit stark basischen Lösungen und die Sterilisation mittels Autoklavieren bei Temperatur bis zu 135° C und typischen Dampfdrücken von etwa 3 bar zu nennen. Typischerweise geht man dann von einigen Zehn bis mehreren Hundert derartiger Aufbereitungszyklen aus. Dies erfordert hohe Ansprüche an die thermische, chemische und auch hydrolytische Beständigkeit.

Bei der EVLT führt der behandelnde Arzt über eine winzige Punktionsstelle einen Katheder in die betroffene Vene ein, der als Leitschiene für den Venenlaser dient. Durch seitliche Abstrahlung der Laserenergie mittels des Diffusors wird die Gefäßinnenwand anschließend stark erwärmt, wodurch die Vene kollabiert und verschlossen wird. Der krankhafte Rückfluss des venösen Blutes wird somit unterbunden. In der Folge verhärtet die Vene, bildet sich zurück und kann vom Körper abgebaut werden. In der Regel werden sogenannte Ring- oder Doppelring-Fire-Systeme als Lichtquelle verwendet. Dabei wird das Abstrahlelement zur gleichmäßigen Behandlung oft manuell mit möglichst konstanter Geschwindigkeit durch den zu behandelnden Venenabschnitt gezogen, was die Applikation erschwert, da bei Nichtbeachtung beziehungsweise zu langer Verweildauer an einer Stelle weitere Zellschädigungen entstehen können.

Bei der LITT handelt es sich um ein minimal-invasives Verfahren, das zur lokalen Tumordestruktion eingesetzt wird. Dabei wird unter bildgebender Kontrolle (zum Beispiel Sonographie / MRT) der Tumor punktiert, eine (oder mehrere) Laserfaser(n) in den Tumorherd eingebracht und dieser durch thermische Energie verödet. Zum Einsatz kommen hier insbesondere Nd:YAG-Laser (1064 nm) sowie Diffusor-Tip-Applikatoren. Die Laserleistung liegt bei etwa 5 bis 8 W (s. u.a. "Laserinduzierte Interstitielle Thermotherapie (LITT) bei malignen Tumoren", BÄK und KBV 01/2002).

Ein weiterer, insbesondere volumenstreuender Diffusor ist beispielsweise in EP 3184885A1 beschrieben. Diese beschreibt einen Diffusor am Ende einer Lichtleitfaser aus Quarzglas, wobei zur Erzeugung des Diffusors vorgesehen ist, auf dem distalen Faserende der Lichtleitfaser eine Streumasse aufzubringen und diese zu dem Diffusor zu verfestigen. Nachteilig bei derartigen Ansätzen ist, dass diese volumenstreuenden Ansätze einen stark exponentiellen Abfall der Intensität mit sich bringen. Auch sind poröse Materialien mit Blick auf deren Aufbereitbarkeit in medizintechnischen Anwendungen nicht bevorzugt.

In der Schrift US 6,810,184 B2 wird ein Ansatz beschrieben, bei dem nanoporöse Siliziumdioxid-plattierte optische Fasern verwendet werden, um Fasern mit integral gebildeten Diffusionspitzen herzustellen, die mit anderen Fasern verschmolzen werden können. EP 2062077 A4, US 2009/0204111 A1 und DE 102015119875 A1 beschreiben Diffusoren, bei denen zur ihrer Erzeugung mittels Laser in, beziehungsweise auf der Faser Strukturen ein-, beziehungsweise aufgebracht werden.

WO 2008/024397 A2 stellt u.a. einen Diffusor zum Ausgeben von optischer Energie hoher Leistungsdichte zu einer Behandlungsstelle am distalen Ende mindestens einer optischen Faser vor. Dabei ist vorgesehen, dass Streuungszentren in der vorbestimmten Länge des Faserkerns oder in oder nahe einer Grenzfläche zwischen dem Faserkern und der Umhüllung in der vorbestimmten Länge angeordnet sind.

Bei den vorstehend vorgestellten Ansätzen ist allerdings insbesondere mit den Nachteilen zu rechnen, dass, bei ausreichend homogener Ausgestaltung der Streuzentren, aufgrund des exponentiellen Abfalls der seitlichen Emission oder ungleichmäßiger Verteilungen eine seitliche Abstrahlung nicht mit der im medizinischen Umfeld geforderten Homogenität erzielt wird.

US 2009/0204111 A1 beschreibt ein Laser Delivery System mit einer optischen Faser mit einem Kern und einer Mantelschicht, die mindestens einen Teil des Kerns bedeckt, wobei die Mantelschicht einen niedrigeren Brechungsindex als der Kern aufweist, und einen Nicht-Merkmalsabschnitt und einen Merkmalsabschnitt mit Merkmalen, die das Licht dazu zwingen, sich radial von dem Merkmalabschnitt auszutragen und ein gewünschtes radiales Lichtausgangsmuster zu schaffen. Dabei ist vorgesehen, dass die Merkmale aus der Gruppe ausgewählt sind, die aus spiralförmigen Strukturen, radialen Schnitten, axialen Schnitten und einer Kombination davon besteht.

DE 102015119875 A1 beschreibt einen Lichtwellenleiter umfassend einen lichtwellenführenden Kern, einen Bereich im Lichtwellenleiter, wobei in dem Bereich des Lichtwellenleiters Mikromodifikationen angeordnet sind und wobei die Anordnung der Mikromodifikationen geordnet ist.

WO 2019/063799 A1 beschreibt ein Beleuchtungssystem insbesondere für ein medizintechnisches Therapie- und/oder Diagnosesystem, welches wenigstens eine Laser-Lichtquelle, einen Lichtleiter, der an einem proximalen Ende an die wenigstens eine Laser-Lichtquelle anschließbar und/oder dieser zuordenbar ist, und welches am distalen Ende des Lichtleiters ein Diffusor-Element mit einer Längsachse aufweist, die senkrecht zu einer Einkoppelfläche des Lichtleiters in das oder in dem Diffusor-Element verläuft, umfasst. Hierbei strahlt das Diffusor-Element Licht über seine aktive Länge seitlich zur Längsachse ab, wobei das Diffusor-Element mindestens einen Diffusor-Grundkörper aufweist und der Diffusor-Grundkörper mindestens ein Streuelement beinhaltet. Dabei weist das Beleuchtungssystem eine Einrichtung zur Homogenisierung der Abstrahlungsintensität entlang der Längsachse des Diffusor-Grundkörpers auf, wobei das Beleuchtungssystem im Betriebszustand eine Intensitätsverteilung der seitlichen Abstrahlung aufweist, die um höchstens ± 50%, bevorzugt höchstens ± 30% und meist bevorzugt höchstens als ± 5% von der mittleren seitlichen Abstrahlungsintensität abweicht. Daher können insbesondere sehr homogen über die Länge abstrahlende Zylinder-Diffusoren realisiert werden, die zudem eine hohe Effizienz und eine geringe Eigenerwärmung aufweisen, was insbesondere für die Anwendungen PIT und PDT vorteilhaft ist.

Bei vielen Anwendungen im medizintechnischen Umfeld kann, wie zuvor erwähnt auch gesundes Gewebe bestrahlt und somit geschädigt werden, welches meist direkt benachbart an dem zu bestrahlenden Gewebe angrenzt. Es wird daher angestrebt, dieses Gewebe zukünftig vor einer ungewünschten Bestrahlung zu schützen.

EP 0 761 257 A2 beschreibt eine Sonde zur Laserbestrahlung, die in einem medizinischen Lasergerät zur Durchführung einer Laserbehandlung durch Bestrahlung eines Menschen oder eines Tieres mit Laserlicht oder -strahlen verwendet wird. Die Lasersonde umfasst insbesondere einen Laserlichtreflektor, der zwischen dem transparenten Lichtdiffusor und einem schützenden Diffusionsrohr vorgesehen ist.

WO 2017/174549 A1 beschreibt ein Bestrahlungssystem mit einer Strahlungsquelle, einer Einkoppeleinheit zum Einkoppeln der Strahlung der Strahlungsquelle in einen vorderen Endbereich eines Lichtwellenleiters zur Übertragung der Strahlung zu einer an einem hinteren Endbereich des Lichtwellenleiters vorgesehenen Auskoppeleinheit. Das Bestrahlungssystem ist vorgesehen zur Bestrahlung von Behandlungsgut in industriellen Prozessen zur Erwärmung, Polymerisation, Aushärtung oder/und Desinfektion mit IR- und/oder UV-Strahlung.
US 4 466 697 A beschreibt optische Elemente mit Kernstrukturen zur Lichtemission oder - übertragung. Eine optische Faser umfasst einen inneren Kern aus lichtleitendem Kunststoff oder Glas, der mit einer äußeren Mantelschicht versehen ist. Die Mantelschicht kann eine Defektstelle aufweisen an welcher eine Lichtemission bewirkt wird.

Aufgabe der Erfindung ist es daher, eine verbesserte Bestrahlung von ungesundem Gewebe zu ermöglichen, welche die besonderen Anforderungen in einem medizintechnischen Umfeld erfüllt und insbesondere gesundes Gewebe schützt, sodass unerwünschte körperliche Schädigungen an Personen vermieden werden.

Diese Aufgabe wird durch den Gegenstand der unabhängigen Ansprüche gelöst. Vorteilhafte Weiterbildungen sind in den jeweiligen abhängigen Ansprüchen angegeben.

Demgemäß betrifft die Erfindung ein Beleuchtungssystem für ein medizintechnisches Therapie- und/oder Diagnosesystem, insbesondere zur Anwendung an lebendem Gewebe, welche wenigstens eine Lichtquelle, einen Lichtleiter, der an einem proximalen Ende an die Lichtquelle anschließbar oder dieser zuordenbar ist, und ein optisches Element, welches bevorzugt als Diffusor-Element ausgebildet ist und an einem distalen Ende des Lichtleiters angeordnet ist, so dass Licht aus dem Lichtleiter in das optische Element einkoppelbar ist, aufweist. Unter einem Diffusor-Element wird im Sinne der Erfindung ein Lichtstreukörper verstanden, insbesondere ein Körper, welcher Licht leitet und vorzugsweise in unterschiedliche Richtungen abstrahlen kann. Dabei kann das Licht diffus oder gerichtet abstrahlbar sein, insbesondere in einer Richtung, welche quer, diagonal, senkrecht oder parallel zu der Richtung verläuft, in welche das Licht in das optische Element eingekoppelt wird.
Das optische Element weist einerseits einen Licht-reflektierenden Bereich auf und andererseits einen Licht-durchlässigen Bereich auf, wobei der Licht-reflektierende Bereich eine zumindest partielle Reflektivität aufweist und der Licht-durchlässige Bereich eine zumindest partielle Transmission, insbesondere eine hohe spektrale Transmission, vorzugsweise bis zu einer Wellenlänge von etwa 2,5 µm aufweist. An dem Licht-reflektierenden Bereich ist Licht zumindest teilweise reflektierbar, welches innerhalb des optischen Elements eingekoppelt ist. Die Reflektion kann dabei diffus oder auch gerichtet abstrahlend sein. An dem Licht-durchlässigen Bereich ist Licht, insbesondere das zuvor genannte reflektierte Licht, zumindest teilweise aus dem optischen Element auskoppelbar. Insbesondere weist der Licht-reflektierende Bereich eine höhere Reflektivität auf, als der Licht-durchlässige Bereich. Besonders bevorzugt ist die Reflektivität des Licht-reflektierenden Bereichs zumindest 30, insbesondere zumindest 50, insbesondere zumindest 70, Prozentpunkte höher als diejenige des Licht-durchlässigen Bereichs. Vorzugsweise ist dazu das optische Element zumindest abschnittsweise mit mindestens einer Reflektorschicht bedeckt, insbesondere im Bereich des Licht-reflektierendes Bereichs mit einer Reflektorschicht bedeckt, um dem Licht-reflektierenden Bereich eine zumindest partielle Reflektivität zu verleihen. Vorzugweise bleibt das optische Element zumindest abschnittsweise von der Reflektorschicht frei, insbesondere im Bereich des Licht-durchlässigen Bereichs von der Reflektorschicht frei, um dem Licht-durchlässigen Bereich eine zumindest partielle Transmissivität zu verleihen. Die Reflektorschicht kann eine Oberfläche und/oder eine Mantelfläche des optischen Elements bedecken. Ferner ist erfindungsgemäß vorgesehen, dass die Reflektorschicht eine Spiegelschicht umfasst.
Das optische Element kann demnach eine Mantelfläche aufweisen, welche zumindest abschnittsweise von einer Licht-reflektierenden Reflektorschicht bedeckt ist, die vorzugsweise eine Spiegelschicht aufweist, wobei das optische Element einen Licht-reflektierenden Bereich aufweist, welcher von der Reflektorschicht bedeckt ist, und einen Licht-durchlässigen Bereich aufweist, welcher vorzugsweise von der Reflektorschicht frei bleibt, sodass das vom optischen Element abzustrahlende Licht zumindest teilweise reflektierbar ist, und Licht an dem Licht-durchlässigen Bereich abstrahlbar ist. Insbesondere kann hiermit Licht gezielt an dem Licht-durchlässigen Bereich abstrahlbar sein. Unter dem Begriff der Mantelfläche ist insbesondere eine Oberfläche des optischen Elements zu verstehen.
Erfindungsgemäß ist die Reflektivität der Reflektorschicht für zumindest einen Wellenlängenbereich größer als 90%. Die Reflektorschicht ist erfindungsgemäß als Spiegelschicht ausgebildet.
Der Wellenlängenbereich, für den die Reflektorschicht ausgelegt ist, kann vorzugsweise den sichtbaren Spektralbereich (VIS) zwischen ca. 400 nm und ca. 700 nm, z.B. insbesondere z.B. zwischen 400 nm und 450 nm oder zwischen 600 nm und 700 nm, als auch den Infrarot-Bereich (IR) zwischen ca. 700 nm und ca. 2,5 µm, insbesondere den nahen Infrarot-Bereich (NIR) zwischen ca. 700 nm und ca. 1200 nm, z.B. 980 nm oder 1064 nm, umfassen. Denkbar sind auch Wellenlängen im nahen UV-Bereich zwischen ca. 350 nm und ca. 400 nm.
Der Licht-durchlässige Bereich oder Licht-reflektierenden Bereich kann allerdings auch so ausgelegt sein, dass ein vorgebbarer Wellenlängenbereich oder eine vorgebbare Hauptwellenlänge transmittiert, beziehungsweise reflektiert wird. Vorzugsweise wird dieser vorgebbare Bereich um nicht mehr als 20 nm überschritten, bevorzugt nicht mehr als 10 nm, bevorzugt nicht mehr als 5 nm. Ein solcher, vorgebbarer Bereich kann beispielsweise Wellenlängen einer bestimmten Farbe umfassen, insbesondere Grün oder Rot.
Indem das optische Element einerseits einen zumindest partiell Licht-reflektierenden Bereich aufweist und andererseits einen zumindest partiell Licht-durchlässigen Bereich aufweist, wobei der Licht-reflektierende Bereich eine höhere Reflektivität aufweist, als der Licht-durchlässige Bereich, kann in vorteilhafter Weise erreicht werden, dass der Lichtaustritt an dem Licht-reflektierenden Bereich vermindert oder vermieden wird, während der Lichtaustritt an dem Licht-durchlässigen Bereich erhöht oder auf diesen beschränkt wird. Hierdurch kann in vorteilhafter Weise eine gezieltere Lichtabgabe ermöglicht werden, insbesondere um krankes Gewebe zu bestrahlen und zugleich benachbartes gesundes Gewebe zu schützen.
Insbesondere durch die nur abschnittsweise auf das Diffusor-Element aufgebrachte Reflektorschicht wird das Licht gezielt an unbedeckten Bereichen abgegeben, wodurch während der medizinischen Behandlung gezielt, beispielsweise nur krankes oder zu bestrahlendes Gewebe bestrahlt werden kann, beispielsweise Gewebe durch Energieeintrag gezielt verödet wird, beispielsweise im Rahmen einer LITT-Anwendung. Angrenzendes, gesundes Gewebe wird auf diese Weise vor der Bestrahlung geschützt und wird idealerweise nicht geschädigt.
In einer Ausführungsform ist das Licht quer zu einer Längsachse des optischen Elements und seitlich über eine aktive Länge des optischen Elements abstrahlbar, beziehungsweise auskoppelbar. Wenn das optische Element als Lichtwellenleiter ausgebildet ist, kann es vorteilhafterweise Licht in eine vorgegebene Richtung, beispielsweise entlang der Längsachse leiten. Als seitliche Abstrahlung wird im Rahmen der vorliegenden Offenbarung eine Abstrahlung verstanden, welche Richtungsanteile aufweist, die in radialer Richtung ausgehend von der Längsachse des Diffusor-Elements verlaufen. Als seitliche Abstrahlungsintensität wird die Intensität dieser Abstrahlung verstanden.
Die Geometrie der Abstrahlung kann dabei durch eine entsprechende geometrische Anpassung der Reflektorschicht angepasst werden, so dass beispielsweise streifenförmige oder auch linsenförmige Muster auf die zu behandelte Gewebeoberfläche projiziert werden können. Vorteilhaft ist demnach, wenn sich zumindest ein, von der Reflektorschicht unbedeckter Bereich des Diffusor-Elements, beispielsweise eine Aussparung der Reflektorschicht über die Längsrichtung des Diffusor-Elements erstreckt und dieser unbedeckte Bereich lichtdurchlässig ist, so dass durch das Diffusor-Element geleitetes Licht streifenförmig, insbesondere linienförmig abgebbar ist. Der lichtdurchlässige Bereich kann dabei linear oder auch nicht linear verlaufen. Ein linienförmiger Abstrahlbereich ermöglicht eine sektionale Abstrahlung, so dass das zu bestrahlende Gewebe nicht punktförmig, sondern in einem größeren Bereich bestrahlt werden kann und gleichzeitig dieser Bereich in definierten Grenzen gehalten werden kann, um umliegendes Gewebe zu schützen.
Durch entsprechende geometrische Anpassung der Reflektorschicht, gegebenenfalls auch in Kombination mit der Geometrie des optischen Elementes, lassen sich auch komplexere Abstrahlgeometrien erzielen. So sind beispielsweise auch graduelle Bereiche möglich, wobei die Reflektorschicht in diesem Fall in einem definierten Bereich schwächer, insbesondere mit geringerer Reflexionsleistung, ausgeprägt sein kann, als in einem stark reflektierenden Bereich. Denkbar sind allerdings auch Geometrien, bei denen sich beispielsweise ein lichtdurchlässiger, insbesondere streifenförmiger Bereich in einer Richtung, beispielsweise eine Längsrichtung verbreitert oder verschmälert. Dabei kann sich ein, beispielsweise streifenförmiger, linsenförmiger oder ovaler Bereich der Länge nach entlang, quer und/oder auch diagonal der/zur Längsachse des optischen Elements erstrecken.

Idealerweise ist das Diffusor-Element länglich, beziehungsweise stabförmig ausgebildet, sodass es bspw. in einen Katheter eingeschoben werden kann. Das Diffusor-Element kann weiterhin einen im Wesentlichen kreisrunden, ovalen oder eine polygonen, beispielsweise sechseckigen, rechteckigen oder quadratischen Querschnitt aufweisen, auch sternformige Formen können realisiert werden. Das Licht wird in diesen Fällen vorzugsweise seitlich über die Facetten, beziehungsweise radiale Flächen abgestrahlt. Die nur abschnittsweise oder facettenweise auf das Diffusor-Element aufgebrachte Reflektorschicht ermöglicht es demnach, Licht seitlich nur eine bestimmte Richtung, beziehungsweise in eine definierte Sektion abzustrahlen. Besonders vorteilhaft ist es, wenn zumindest eine oder mehrere Facetten eine linsenförmige Form aufweisen, vorzugsweise die Form einer Fresnel-Linse, sodass das Licht gebündelt werden kann und insbesondere eine intensivere Bestrahlung, beziehungsweise höherenergetische Bestrahlung ermöglicht wird.

Das Diffusor-Element und/oder optische Element weist wie beschrieben vorzugsweise eine längliche, stabförmige oder zylindrische Gestalt auf, so dass das Diffusor-Element und/oder optische Element eine Längsachse definiert. Die Mantelfläche ist somit erfindungsgemäß eine um die Längsachse verlaufende Mantelfläche. Insbesondere steht der Normalenvektor der Mantelfläche somit senkrecht zu der Längsachse.

Es kann vorgesehen sein, dass der Licht-reflektierende Bereich des optischen Elements und/oder Diffusor-Elements, welcher wie beschrieben von der Reflektorschicht bedeckt ist, sich tangential zur Längsachse zumindest über einen bestimmten Winkelbereich erstreckt (z.B. über zumindest 90° oder 180° oder 270°) und der Licht-durchlässige Bereich, welcher wie beschrieben vorzugsweise von der Reflektorschicht frei bleibt, sich ebenfalls tangential zur Längsachse zumindest über einen bestimmten Winkelbereich erstreckt (z.B. über zumindest 1° oder 10° oder 20°). Der Licht-durchlässige Bereich kann somit z.B. als ein tortenstückförmiger Teil der Mantelfläche ausgebildet sein, aber selbstverständlich auch komplexere Geometrien aufweisen wie bereits beschrieben.

Die Reflektorschicht ist insbesondere konzentrisch auf zumindest einem Teil der Mantelfläche oder aber auf zumindest einer Facette aufgebracht. Dabei ist die Reflektorschicht vorzugsweise derart ausgebildet, dass Licht aus dem Diffusor-Element wieder zurück in die Matrix des Diffusor-Element reflektierbar ist und/oder von außen kommendes Licht wieder nach außen zurückreflektiert wird. Das bedeutet auch, dass die Reflektorschicht nur einseitig reflektierbar sein kann, sodass von außen kommendes Licht in das Diffusor-Element eingeleitet werden kann, die Reflektorschicht also transmissiv für Licht von außen ist. Die Reflektorschicht und/oder die Spiegelschicht kann demnach also als dichroitischer Spiegel ausgebildet sein, beispielsweise als Kurzpass-, Langpass-, Schmalband- oder Breitband-Spiegel.

Vorzugsweise ist die maximale Reflektivität größer als 95 % und besonders bevorzugt größer als 99 %, insbesondere bei senkrechtem Lichteinfall auf die Reflektorschicht und die Reflektivität ist gezielt auf eine definierte Wellenlänge des verwendeten Lichts oder in einem vorgebbaren Bereich um eine Haupt-Wellenlänge des Lichts einstellbar ist.

In einer vorteilhaften Ausführungsform ist die Reflektivität der Reflektorschicht bei einem Lichteinfallswinkel von größer als 45°, bevorzugt größer als 60°, besonders bevorzugt größer als 80° bezogen auf die Senkrechte zur Reflektorschicht größer, als 50% bevorzugt 70%, meist bevorzugt 90% der Reflektivität bei senkrechtem Einfall, insbesondere in einem Bereich um ein Maximum der Reflektivität bei senkrechtem Lichteinfall. Mit anderen Worten kann die Reflektivität in einem Bereich, insbesondere in einem Winkelbereich um die maximale Reflektivität bei senkrechtem Lichteinfall, vorzugsweise dem zuvor angegeben Winkelbereich größer sein als 50%, bevorzugt größer als 70%, bevorzugt größer als 90%.
Vorzugsweise wird Licht in einem vorgegebenem Wellenlängenbereich durch das Diffusor-Element geleitet. Unterschiedliche Wellenlängen besitzen jedoch eine unterschiedliche Reflektivität. Vorteilhafterweise wird daher eine Reflektorschicht verwendet, welche eine besonders hohe Reflektivität für verschiedene Wellenlängen und/oder eine für großen Winkelbereich aufweist, so dass bei dem verwendeten Licht mehrere Maxima bzw. ein wellenlängenabhängiges breites Reflektionsmaximum-Plateau auftritt und die entsprechenden Wellenlängen auch bei schrägem Lichteinfall eine hohe Reflexion, insbesondere größer 90% aufweisen.

Die Reflektivität kann daher auf die verwendeten Wellenlängen und Winkel eingestellt werden. Auf diese Weise kann eine besonders hohe Menge des Lichts reflektiert werden. Dies ist besonders wichtig, da eine Absorption von Licht vermieden werden soll. Da das Beleuchtungssystem mit Leistungen bis zu 30 Watt, typischerweise mit Leistungen zwischen 10 W und 20 W, betrieben werden kann, kann es durch Absorption zu einer starken Erwärmung des Diffusor-Elements und/oder der Reflektorschicht kommen. Eine derartig hohe Absorption kann zur Schädigung des Beleuchtungssystems durch, beispielsweise Überhitzung führen, oder in schlimmeren Fällen sogar zu Verletzungen an Personen. Das Beleuchtungssystem soll also durch hohe Reflexion im Betrieb so kalt gehalten werden, dass es sich nicht selbst schädigt. Mit anderen Worten, es soll eine Eigenerwärmung des optischen Elements, beziehungsweise des Diffusor-Elements vermieden werden, vorzugsweise dadurch, dass eine maximale Menge Licht reflektiert wird.

Es ist auch denkbar, dass die Spiegelschicht, insbesondere der Reflektorschicht eine metallische Schicht aufweist oder aus dieser ausgebildet ist, welche vorzugsweise ein oder mehrere Metalle aus der Gruppe der Edelmetalle oder ein Metall der Gruppe aus Mg, Al, Cu umfasst. Auch eine Legierung dieser Materialien ist denkbar. Aufgrund der hohen Reflektivität in einem großen Wellenlängenbereich werden bevorzugt Edelmetalle, wie Au, Pt, Pd oder Ag verwendet. Auch deren Verarbeitbarkeit im Hinblick auf die Herstellung der Reflektorschicht ist vergleichsweise einfach. Aufgrund der geringen Oxidationsfähigkeit von Au, ist es inert, beispielsweise gegenüber Körperflüssigkeiten und weist darüber hinaus eine geringe Wechselwirkung mit körpereigenen Proteinen auf, sodass die Biokompatibilität von Au besonders gut ist und daher bevorzugt wird. Insgesamt soll die Reflektorschicht den Anforderungen, insbesondere standardisierten Anforderungen der Biokompatibilität und Zytotoxizität, wie sie in der Medizin gefordert sind, entsprechen.
Wie beschrieben ist die Mantelfläche des Diffusor-Elements bzw. optischen Elements zumindest abschnittsweise von einer Reflektorschicht bedeckt, welche vorzugsweise eine Spiegelschicht umfasst. In einer Weiterbildung kann die Reflektorschicht mehrschichtig und/oder als Schichtsystem ausgebildet sein. In diesem Fall kann z.B. die Spiegelschicht der Reflektorschicht aus mehreren Schichten bestehen. Eine mehrschichtige Reflektorschicht kann aber auch eine oder mehrere weitere Schichten umfassen, welche zusätzlich zu der Spiegelschicht vorhanden sind, insbesondere unterhalb und/oder oberhalb der Spiegelschicht angeordnet sind. Die an anderen Stelle näher definierte bevorzugte Reflektivität der Reflektorschicht bezieht sich im Fall einer mehrschichtigen bzw. als Schichtsystem ausgebildeten Reflektorschicht auf die gesamte Reflektorschicht, d.h. umfassend die aus einer oder mehreren Schichten bestehende Spiegelschicht sowie die aus einer oder mehreren Schichten bestehenden weiteren Schichten.
Alternativ oder zusätzlich ist es aber selbstverständlich auch möglich, dass unterhalb und/oder oberhalb der Reflektorschicht nochmals weitere Schichten vorgesehen sind, welche nicht zu der Reflektorschicht gehören.

Erfindungsgemäß ist vorgesehen, dass die Reflektorschicht mit mindestens einer Spiegelschicht als Schichtsystem ausgebildet ist. Es kann vorgesehen sein, dass die Reflektorschicht mindestens eines der folgenden Merkmale aufweist:
- Unterhalb der Reflektorschicht ist ein Grundschichtbereich bestehend aus einer einzelnen Schicht oder einer Abfolge von Schichten vorgesehen,
   - zwischen einem Diffusor-Grundkörper und der Spiegelschicht ist mindestens eine Haftschicht oder Haftvermittlerschicht vorgesehen,
   - zwischen dem Diffusor-Grundkörper und der Spiegelschicht ist ein Schichtbereich auf dem Diffusor-Grundkörper mit chemisch und/ oder physikalisch veränderten Hafteigenschaften vorgesehen,
   - die Reflektorschicht weist in einem nach außen gerichteten Bereich mindestens eine Passivierungsschicht auf, welche die Reflektorschicht zumindest teilweise abdeckt.
Mit anderen Worten kann ein Mantel-Reflektor des Beleuchtungssystems mit seiner mindestens einen Reflektorschicht als Schichtsystem ausgebildet sein, und/oder zwischen einem Diffusor-Grundkörper, welcher auch als Diffusor-Element bezeichnet wird, und der Reflektorschicht kann mindestens eine Haftschicht oder Haftvermittlerschicht vorgesehen sein und/oder ein Schichtbereich auf dem Diffusor-Grundkörper mit chemisch und/oder physikalisch veränderten Hafteigenschaften.
Erfindungsgemäß ist vorgesehen, dass die Reflektorschicht eine Spiegelschicht aufweist und zusätzlich zu der Spiegelschicht eine unterhalb der Spiegelschicht befindliche untere Schicht aufweist, und wobei die Reflektivität der Reflektorschicht für zumindest einen Wellenlängenbereich größer als 90% ist.
Wie beschrieben kann in einer Weiterbildung die Reflektorschicht mehrschichtig bzw. als Schichtsystem ausgebildet sein, wobei insbesondere vorgesehen sein kann, dass die Reflektorschicht zusätzlich zu der Spiegelschicht noch zumindest eine weitere Schicht umfasst.
Demnach kann die Reflektorschicht zusätzlich zu der Spiegelschicht eine unterhalb der Spiegelschicht befindliche untere Schicht und/oder eine oberhalb der Spiegelschicht befindliche obere Schicht aufweisen, wobei die untere Schicht insbesondere aus einer oder mehreren Schichten besteht und wobei die obere Schicht insbesondere aus einer oder mehreren Schichten besteht.
Eine unterhalb der Spiegelschicht befindliche untere Schicht ist vorzugsweise ausgebildet als Haftschicht oder Haftvermittlerschicht.
Eine unterhalb der Spiegelschicht befindliche untere Schicht ist vorzugsweise ausgebildet ist als Schichtbereich auf dem Diffusor-Grundkörper mit chemisch und/oder physikalisch veränderten Oberflächeneigenschaften.
Eine unterhalb der Spiegelschicht befindliche untere Schicht ist vorzugsweise ausgebildet als ein Grundschichtbereich bestehend aus einer einzelnen Schicht oder einer Abfolge von Schichten. Ein Grundschichtbereich, z.B. ein unterhalb der Spiegelschicht befindlicher Grundschichtbereich, welcher insbesondere Teil der Reflektorschicht ist, und/oder ein unterhalb der Reflektorschicht vorgesehener Grundschichtbereich kann demnach eine oder mehrere auf dem optischen Element aufgebrachte Schichten umfassen. Es kann alternativ oder zusätzlich vorgesehen sein, dass der Grundschichtbereich eine oberflächliche Schicht des optischen Elements mit einer veränderten Oberflächeneigenschaft umfaßt, insbesondere mit einer erhöhten Oberflächenenergie und/oder einer erhöhten Anzahl von Sauerstoffradikalen, insbesondere hergestellt oder herstellbar mittels eines chemischen und/oder physikalischen Prozesses zur Veränderung zumindest einer Oberflächeneigenschaft des optischen Elements. Der Grundschichtbereich, z.B. der unterhalb der Spiegelschicht befindliche Grundschichtbereich, welcher insbesondere Teil der Reflektorschicht ist, und/oder der unterhalb der Reflektorschicht vorgesehene Grundschichtbereich kann insbesondere die Haftschicht oder die Haftvermittlerschicht bilden.

Es ist daher möglich, dass die Reflektorschicht oder Spiegelschicht direkt, also insbesondere ohne eine Haftschicht auf dem Diffusor-Element angeordnet ist. Dabei kann, muss aber nicht, auf der Reflektorschicht und/oder der Spiegelschicht die Passivierungsschicht angeordnet sein. Es kann daher auch vorgesehen sein, das Diffusor-Element, beziehungsweise die Reflektorschicht sowohl mit, als auch ohne Haftschicht und/oder Passivierungsschicht auszubilden. Die Haftschicht, beziehungsweise der Schichtbereich mit veränderten Hafteigenschaften sorgt für eine verbesserte Haftung der Reflektorschicht auf dem optischen Element beziehungsweise dem Diffusor-Element beziehungsweise dem Diffusor-Grundkörper. Dies ist insbesondere mit Blick auf die Korrosionsbeständigkeit der Reflektorschicht vorteilhaft, wenn das Beleuchtungssystem beispielsweise mehrfach verwendet wird und die Reflektorschicht aufwendige Reinigungs- und Sterilisationsprozesse überstehen soll, bei denen ätzende Säuren und/oder Basen eingesetzt werden. Auch mit Blick auf die Abriebfestigkeit ist eine verbesserte Haftung vorteilhaft, da beispielsweise während der Bewegen des Beleuchtungssystems innerhalb eines Katheters Reibung entstehen kann und die Reflektorschicht zuverlässig am Diffusor-Grundkörper haften bleibt. Es ist aber auch denkbar, dass die Haftschicht derart ausgebildet ist, dass sie einen positiven Beitrag zur Reflexion leistet. Mit anderen Worten, es ist vorgesehen, dass das Material, die Oberfläche und/oder die Kristallorientierung der Haftschicht gegenüber den Eigenschaften des optischen Elements oder des Diffusor-Elements, so ausgewählt ist, dass zumindest ein Teil des Lichts oder vorbestimmte Wellenlängen reflektiert werden.
Erwähnenswert ist demnach, dass eine bevorzugte Reflektivität einer Reflektorschicht, welche zusätzlich zu der Spiegelschicht noch eine oder mehrere weitere Schichten umfasst, also z.B. einen Grundschichtbereich und/oder eine Passivierungsschicht umfasst, sich auf die gesamte Reflektorschicht bezieht. Insbesondere können unterhalb der Spiegelschicht auf dem optischen Element aufgebrachte Schichten und/oder oberhalb der Spiegelschicht aufgebrachte Schichten zu der Reflektivität der Reflektorschicht beitragen. Dies gilt insbesondere auch für eine unterhalb der Spiegelschicht befindliche oberflächliche Schicht des optischen Elements, welche z.B. mittels chemischer und/oder physikalischer Prozesse herstellbar ist. Denn durch diese Prozesse erfolgt eine Veränderung im Material des optischen Elements innerhalb einer oberflächlichen Schicht des optischen Elements, welche daher dann einen Teil Reflektorschicht bilden kann.

Es kann auch vorgesehen sein, dass das Schichtsystem im äußeren Bereich zusätzlich mindestens eine Passivierungsschicht aufweist, welche die Reflektorschicht vollständig abdeckt. Die Passivierungsschicht bietet zusätzlichen Schutz, beispielsweise vor Säuren oder anderen potentiell korrosiven Medien, wie Gasen oder Flüssigkeiten. Zudem kann die Passivierungsschicht die Biokompatibilität der Reflektorschicht gegenüber Substanzen des biologischen Körpers beziehungsweise Materials in dem das Beleuchtungssystem verwendet werden soll verbessern, sodass auch ein/e zu behandelnde/r Patient/in, geschützt werden kann.

Es ist auch vorgesehen, dass das Beleuchtungssystem zumindest eines der folgenden Merkmale aufweist:
- die untere Schicht (43.1), insbesondere die Haftschicht oder Haftvermittlerschicht oder der Grundschichtbereich, ist als dielektrische Schicht ausgebildet, wobei die dielektrische Schicht vorzugsweise Oxide, Nitride oder Oxinitride aus mindestens einem Element der Gruppe aus Si, Al, Ti, Zr, Hf, Y, Zn aufweist,
- die obere Schicht (43.2), insbesondere die Passivierungsschicht (43.3) ist als dielektrische Schicht ausgebildet, wobei die dielektrische Schicht vorzugsweise Oxide, Nitride oder Oxinitride aus mindestens einem Element der Gruppe aus Si, Al, Ti, Zr, Hf, Y, Zn aufweist.
Derartige Materialien eignen sich in besonderem Maße als Haftvermittler von insbesondere Schichten aus reinen Metallen, beispielsweise auf Glas. Zudem sind Metalloxide und Metallnitrite sehr korrosisonsresistent gegenüber chemischen und/oder mechanischen Belastungen, beispielsweise Abrieb. Insbesondere Oxide des Ti und Zr weisen eine geringe Wechselwirkung zu Körperflüssigkeiten auf, was zu einer hohen Biokompatibilität führt. Daher sind derartige Substanzen in besonderem Maße auch als Passivierungsschicht geeignet.

In einer vorteilhaften Ausführungsform, ist die Spiegelschicht oder die Reflektorschicht als ein dielektrisches Mehrschichtsystem ausgebildet, welches eine Abfolge von niedrig- und hochbrechenden Metall-Oxiden und/oder -Nitriten aufweist. Derartige Schichtsysteme sind besonders in Hinblick auf die Reflexionseigenschaften optimal und in großem Maße einstellbar, beziehungsweise anpassbar, sodass das Design, beziehungsweise Schichtsystem, vorzugweise das Beleuchtungssystem mit der Reflektorschicht auf spezielle Anwendungen abstimmbar ist, und insbesondere der zu reflektierende Wellenlängenbereich präzise einstellbar ist.

In einer weiteren Ausführungsform kann auch vorgesehen sein, dass die Spiegelschicht oder die Reflektorschicht als eine Schicht mit Streuzentren ausgebildet ist und auch in Kombination mit Reflektorschichten aus Metallen und/oder dielektrischen Schichten ausgeführt sein kann, wobei die ggf. zusätzlichen Reflektorschichten aus Metallen und/oder dielektrischen Schichten die Schicht mit den Streuzentren außen zumindest bereichsweise umschließt. Insbesondere können streuende Elemente in bestimmter Konzentration bzw. in bestimmter Schichtdicke eine reflektierende Wirkung in einer Vorzugsrichtung erzeugen. Grundsätzlich gilt hierbei, dass die reflektierende Wirkung mit zunehmender Streuzentren-Konzentration und mit der Dicke dieser Schicht mit Streuzentren zunimmt. Beispiele hierfür sind Schichten, welche weiße Farbpigmente aufweisen bzw. Störstellen eingelagert sind, an denen das einfallende Licht gestreut wird. Die ggf. zusätzlichen metallischen und/oder dielektrischen Reflektorschichten können einen Lichtdurch nach außen komplett vermeiden.

Vorteilhaft ist auch, wenn die Reflektorschicht als ein Schichtsystem aus drei, vier oder mehr Schichten ausgebildet ist. Auf diese Weise können einzelne Schichten besser aufeinander abgestimmt werden, und insbesondere die zu reflektierenden Wellenlängen sehr präzise eingestellt werden. Auf diese Weise lässt sich vorteilhafterweise auch der Grad der Reflektivität einstellen, so dass eine besonders hohe Reflektivität des verwendeten Lichts erreicht werden kann. Um die Reflektivität zu erhöhen, kann auch vorgesehen sein, die Spiegelschicht in ein Mehrschichtsystem, insbesondere ein dielektrisches Schichtsystem einzubetten.

Es ist auch denkbar, dass die Schichtdicken der Schichten nach mindestens einem der folgenden Merkmale definiert sind:
- die Dicke der Haftschicht ist größer als 5 nm, bevorzugt größer als 30 nm, und/oder kleiner als 3000 nm, bevorzugt kleiner als 300 nm, bevorzugt kleiner als 150 nm,
- die Dicke der Spiegelschicht ist größer als 10 nm, bevorzugt größer als 20 nm, bevorzugt größer als 50 nm und/oder kleiner als 5000 nm, bevorzugt kleiner als 200 nm, bevorzugt kleiner als 100 nm,
- die Dicke der Passivierungsschicht ist größer als 5 nm, bevorzugt größer als 100 nm, bevorzugt größer als 150 nm und/oder kleiner als 5000 nm, bevorzugt kleiner als 500 nm, bevorzugt kleiner als 250 nm.
Die Haftschicht wird durch eine geringe Schichtstärke derart ausgebildet, dass sie die Reflektivität der Reflektorschicht in möglichst geringem Maße, insbesondere minimal beeinflusst. Dementsprechend wird die Dicke der Spiegelschicht durch die angegebenen Werte so ausgebildet, dass die Transmission von Licht auf ein Minimum verringert wird und gleichzeitig insgesamt möglichst wenig Material verwendet werden muss. Die Schichtdicke der Spiegelschicht und/oder der Reflektorschicht ist beispielsweise derart abgestimmt, dass Wellenlängen oberhalb von 0,35 µm, vorzugsweise oberhalb von 0,4 µm, bevorzugt oberhalb von 0,8 µm und/oder unterhalb von 2,5 µm, vorzugsweise unterhalb von 1,6 µm, vorzugsweise unterhalb von 1,2 µm reflektierbar sind. Die Schichtdicke der Spiegelschicht und/oder der Reflektorschicht ist weiter insbesondere auf eine Anwendungswellenlänge abgestimmt. Je nach Anwendungswellenlänge kann die Reflektivität daher eingestellt werden.
Beträgt die Anwendungswellenlänge beispielsweise 2µm, dann wird die Reflektivität zum Beispiel auf einen Wellenlängenbereich zwischen 1,9µm und 2,1µm oder enger optimiert, bei zum Beispiel 690nm. zwischen 670nm und 710nm. Mit anderen Worten wird die Reflektivität der Spiegelschicht und/oder der Reflektorschicht auf eine Wellenlänge eingestellt, welche höchstens 100nm, bevorzugt 50nm, bevorzugt 20nm von der Anwendungswellenlänge abweicht. Die Dicke der Passivierungsschicht ist so gewählt, dass die Korrosionsbeständigkeit gewährleistet ist.

Sinnvollerweise sollte die Abriebsfestigkeit beziehungsweise die Haftung der Reflektorschicht, wie sie zuvor in verschiedenen Ausführungsvarianten beschrieben ist, zumindest gegenüber üblichen, oder standardisierten Abrasivtests und Haftungstests beständig sein. Durch derartige Tests lässt sich die mechanische, beziehungsweise physikalische Korrosionsbeständigkeit sehr gut verifizieren. Weiterhin kann sichergestellt werden, dass das Beleuchtungssystem im dafür vorgesehenen Umfeld eingesetzt werden darf, da im medizinischen Bereich strenge Regelungen gelten.

Eine gute Haftung, beziehungsweise physikalische Beständigkeit gegenüber mechanischen Einflüssen lässt sich beispielsweise durch einen sogenannten Tape-Test überprüfen. Dabei wird ein Kleberstreifen auf das beschichtete optische Element, beziehungsweise die Reflektorschicht aufgebracht und unter einem definierten Winkel abgezogen. Befindet sich nach dem Abziehen keine Beschichtung an dem Klebestreifen und zeigen sich bei der Beschichtung keine Delaminationen, so gilt der Test als bestanden. Insbesondere ist darauf zu achten, dass auch die gegebenenfalls vorhandene Passivierungsschicht resistent gegenüber der genannten mechanischen Beanspruchung ist.

Eine mechanische Abriebbeständigkeitsprüfung stellt der sogenannte Radiergummi-Test oder auch Rubber-Test dar. Dabei wird ein Radiergummi mit einer bestimmten Kraft einwirkend auf der zu prüfenden Schicht mehrmals hin und her bewegt. Die Schicht gilt dann als abriebsbeständig, wenn nach diesem Testzyklus keine Beschädigungen auf der zu prüfenden Schicht festgestellt werden können.

Die Passivierungsschicht der Reflektorschicht kann auch als Barriereschicht ausgebildet sein. Demnach kann vorgesehen sein, die Passivierungsschicht ein Eindiffundieren von Polymerbestandteilen beispielsweise Säuren und/ oder Sauerstoff und insbesondere von Ionen saurer oder alkalischer Lösungen in die Reflektorschicht, insbesondere an oder in die Spiegelschicht hemmt oder blockiert. Die Passivierungsschicht kann demnach eine Permeation von z.B. Säuren, Sauerstoff oder auch anderen Bestandteilen der Luft in die Reflektorschicht verzögern oder verhindern.

Insbesondere bei der Aufbereitung medizinischer Produkte können alkalische Reiniger bzw. Desinfektoren zum Einsatz (z.B. NEODISHER mit einem pH-Wert von etwa 11) kommen. Zudem sind Sterilisationsverfahren mittels Ethylenoxidgas bekannt, welche insbesondere bei Einwegartiklen (Disposables) eingesetzt werden. Auch hier ist eine chem. Beständigkeit ggü. diesem Gas zu gewährleisten. Im Hinblick auf Autoklavierverfahren (typ. bei 135 °C / 3 bar) muss zudem noch gute hydrolytische Beständigkeit gegeben sein.

Ferner kann eine Spüllösung mit Natriumhypochlorit (NaClO), ein Bleich- oder auch Desinfektionsmittel zum Einsatz kommen, wobei insbesondere die Passivierungsschicht gegenüber derartigen Substanzen zumindest chemisch resistent sein soll. Dies gilt insbesondere für Anwendungen im medizinischen Dental-Bereich.

Vorzugsweise weist die Passivierungsschicht oder die Reflektorschicht auch eine Härte von beispielsweise mindestens 800 HV (Vickers Härte), vorzugsweise mindestens 1200 HV, besonders bevorzugt mindestens 2000 HV gemäß den üblichen, oder standardisierten Prüfverfahren zur Bestimmung der Härte einer Schicht auf. Die Passivierungsschicht kann somit auch als eine mechanische Schutzschicht für die Spiegelschicht oder de Reflektorschicht, insbesondere für eine metallische Spiegelschicht ausgebildet sein. Insbesondere schützen Hartstoffmaterialien aus z.B. Carbiden oder Nitriden aufgrund deren erhöhten Härte, wie z.B. AIN: HV bis ca. 2000, Si3N4: HV bis ca. 2500.

Es ist weiterhin vorgesehen, dass die Reflektorschicht am distalen Ende des Diffusor-Elements angeordnet ist und/oder das Diffusor-Element zumindest teilweise ummantelt. Es versteht sich, dass auch eine am distalen Ende des Diffusor-Elements aufgebrachte Reflektorschicht eine Haftschicht und/oder Passivierungsschicht aufweisen kann. Die Reflektorschicht kann auch als Reflektorfläche verstanden werden, wobei die Reflektorfläche als aufgesputterte oder aufgedampfte dielektrische Reflexionsschichten auf dem distalen Ende des Diffusor-Grundkörpers ausgebildet ist, welche aus mehreren Schichten bestehen und hinsichtlich der Reflektivität auf die Wellenlänge des verwendeten Lichtes abgestimmt sind, wobei vorzugsweise ein Maximum der Reflektivität bei dieser Wellenlänge vorliegt. Idealerweise weist die Reflektorfläche in Form einer gerichtet reflektierenden Fläche beispielsweise, einer metallischen Spiegelflache mit einer Metallbeschichtung, insbesondere umfassend Al, Ag, Au oder einer diffus reflektierenden Fläche, zum Beispiel umfassend eine weiße Farbschicht auf, welche das den Diffusor- Grundkörper passierte Licht in diesen zurück reflektiert. Damit kann der übliche exponentielle Abfall der Intensität des seitlich abgestrahlten Lichtes längs des Diffusor-Grundkörpers zumindest teilweise kompensiert beziehungsweise korrigiert werden. Die bereitstellbare Lichtmenge bei konstanter Streurate ist damit zumindest abschnittsweise verändert beziehungsweise anpassbar, so dass die seitliche Abstrahlung homogenisiert werden kann.

Weiterhin haben sich als besonders vorteilhaft aufgesputterte, oder aufgedampfte dielektrische Reflexionsschichten auf dem distalen Ende des Diffusor-Grundkörpers herausgestellt, welche aus mehreren Schichten bestehen können und hinsichtlich der Reflektivität auf die Wellenlänge des verwendeten Lichtes abgestimmt werden können, dies bedeutet ein Maximum bei der Wellenlange, auf welche abgestimmt wird, aufweisen können. Hiermit kann eine ideale Rückreflektion des im Betriebszustand eingekoppelten Lichtes beziehungsweise dessen Wellenlängen einerseits und eine Vermeidung von Hotspots andererseits erreicht werden. Alternativ kann auch vorgesehen sein, den Reflektor aus einer breitbandig gut reflektierenden Silberschicht mit ruckseitiger Passivierung auszuführen. Diese sind besonders robust und können störende Reflexe unterdrücken, die zu lokalen Intensitätsüberhöhungen und auch Hotspots führen können. Hiermit kann insbesondere ein sehr breitbandiger Reflektor realisiert werden, der sowohl im sichtbaren Spektralbereich (VIS) als auch im IR/MIR-Bereich, beispielsweise zwischen 1 µm und 2,5 µm Wellenlänge, sehr gute Reflexionseigenschaften aufweist. Eine rückseitige Passivierung verhindert eine Oxidation der Silberschicht.

Ist die Reflektorfläche konkav oder konvex ausgebildet, kann erreicht werden, dass reflektierte Strahlen zumindest teilweise mit nahezu parallelem Verlauf zur Längsachse und/oder unter steilerem Winkel zur Längsachse zurückreflektiert und damit an den Streuelementen häufiger gestreut werden, so dass die Auskoppeleffizienz der seitlichen Abstrahlung zum distalen Ende des Diffusor-Elementes hin erhöht wird, was einen homogeneren Verlauf der Abstrahlintensität mit sich bringt.

In einer vorteilhaften Ausführungsform, weist das Diffusor-Element Streuelemente auf, die in die Matrix des Diffusor-Elements eingeschlossen sind, oder die Matrix des Diffusor-Elements von einem Material mit Streuelementen ummantelt ist. Streuelemente sorgen dafür, dass das in das Diffusor-Element eingekoppelte Licht so gestreut, beziehungsweise abgelenkt wird, dass es seitlich aus dem Diffusor-Element austreten kann. Zumindest ein Streuelement ist entlang der kompletten Längsachse des Diffusor-Grundkörpers mit einheitlichem Querschnitt im Wesentlichen zu dieser parallel oder bei sich verjüngenden Diffusor-Grundkörpern in einem Winkel zur Längsachse angeordnet. Das Streuelement kann vorteilhaft auch rohrförmig und insbesondere koaxial zur Langsachse angeordnet sein. Eine Mehrzahl von Streuelementen kann in einer bestimmten vorgebbaren geometrischen Anordnung um die Längsachse des Diffusor-Grundkörpers angeordnet sein, bevorzugt in einer regelmäßigen Struktur um diese, insbesondere bevorzugt kreisförmig. Eine Mehrzahl von unter einem Winkel angeordneten Streuelementen trifft sich somit bevorzugt in einem Fluchtpunkt außerhalb des Diffusor-Grundkörpers.

In einer bevorzugten Ausführungsvariante ist vorgesehen, dass die Streuelemente im Diffusor-Grundkörper radial gleichmäßig verteilt um die Längsachse des Diffusor-Grundkörpers angeordnet sind, wobei eine Kernzone um die Längsachse keine oder eine deutlich reduzierte Anzahl von Streuelementen je Flächeneinheit gegenüber der Anzahl von Streuelementen je Flächeneinheit außerhalb der Kernzone aufweist und somit die Streuelemente überwiegend außerhalb dieser Kernzone in der Matrix angeordnet sind. Damit kann erreicht werden, dass das eingekoppelte Licht, welches in der Regel mit geringer NA (< 0,3, typischerweise um die 0,2) eingekoppelt wird, nicht sofort an den Streuelementen gestreut wird. Andererseits kann durch die nahezu Streu- element-freie Kernzone genügend Licht ohne Streuung bis zum distalen Ende des Diffusor-Grundkörpers geleitet werden. Damit kann einerseits die Intensität nahe der Einkoppelstelle (proximales Ende des Diffusor-Grundkörpers) reduziert und andererseits die Intensität nahe dem distalen Ende Diffusor-Grundkörpers erhöht werden.

Es ist auch denkbar, dass das Diffusor-Element zumindest teilweise oder abschnittsweise in seinem Volumen und/ oder auf seiner Oberfläche strukturiert ist, oder das Diffusor-Element eine eingefärbte oder farblose, insbesondere transparente Ummantelung, vorzugsweise aus einem eingefärbten Glas oder einem eingefärbten Kunststoff aufweist. Eine Struktur auf der Oberfläche des Diffusor-Elements erlaubt eine verbesserte Haftung der Reflektorschicht und/oder verbesserte Streueigenschaften. Beispiele für eine Beschichtung oder Ummantelung, welche zusätzlich eine Lambertsche-Abstrahlcharakteristik unterstutzt und hierbei insbesondere eine in Lichteinkopplungsrichtung vorwärts gerichtete Abstrahlung reduziert, ist eine Beschichtung mit Bornitirid. Weitere Beschichtungen dieser Art können zum Beispiel aus Titanoxid, Kalziumkarbonat, oder Zirkonoxid bestehen. Die zusätzliche Ummantelung kann bspw. als ein Weißglasrohr ausgeführt sein, welches in dessen Glasmatrix streuende Elemente enthält. Eine farblose oder eingefärbte Ummantelung ist allerdings ebenso denkbar.

Eine Ausführungsform sieht daher vor, dass das Diffusor-Element mit der Reflektorschicht zumindest teilweise oder abschnittsweise mit einer transparenten oder transluzenten, farblosen oder eingefärbten Hülle umschlossen ist. Die Hülle hat dabei typischerweise einen Durchmesser, der dem 1,1- bis 1,5-fachen des Durchmessers des Diffusor-Elements entspricht. Bevorzugt ist die Hülle aus einem starren Rohrabschnitt aus Glas oder Metall und/ oder aus einem flexiblen Schlauch gebildet, wobei der Rohrabschnitt und/ oder der Schlauch bevorzugt weitere Streuzentren enthalten kann, und die Hülle zumindest abschnittsweise aus einem oder mehreren dünnwandigen Schrumpfschläuchen ausgeführt ist.

Eine besonders bevorzugte Ausführungsvariante für das Diffusor-Element schlägt vor, dass der Diffusor-Grundkörper distal mit der oben beschriebenen Reflektorfläche mit einer, diesen zumindest teilweise oder abschnittsweise umschließenden transparenten und/oder transluzenten, eingefärbten oder farblosen Hülle versehen ist. Einerseits kann damit ein mechanischer und/oder chemischer Schutz erzielt werden. Andererseits kann durch geeignete Wahl der Materialien, insbesondere, wenn diese Streuzentren beinhalten, auch die Abstrahlcharakteristik hinsichtlich Homogenität der Intensität der seitlichen Abstrahlung weiter optimiert werden. Es kann damit beispielsweise eine Lambertsche Lichtabstrahlung unterstützt werden.

In einer bevorzugten Ausführungsvariante ist die Hülle zumindest abschnittsweise mit einem oder mehreren dünnwandigen Schrumpfschläuchen ausgeführt. Diese können zum einen auch eine zusätzliche diffuse Streuwirkung erzielen und somit eine Lambertsche Abstrahlung unterstützen. Zum anderen kann damit ein mechanischer Schutz erzielt werden und zum Beispiel mögliche Absplitterungen verhindern, falls der Diffusor beschädigt werden sollte. Als Hülle haben sich dafür beispielsweise ein dünnwandiger Schrumpfschlauch aus weiß eingefärbten PET mit einer Wandstärke von etwa 5 bis 15 µm erwiesen. Zur Unterdrückung von Reflexen kann zudem ein dünnwandiger, schwarz oder farbig eingefärbter Schrumpfschlauch partiell vorgesehen sein. Die Einfärbung kann derart gewählt werden, dass die Anwendungswellenlänge besonders gut absorbiert wird. Derartige Schrumpfschlauche sind zudem biokompatibel ausgeführt. Auf diese Weise können Schäden an Personen im Umfeld oder direkter Kontakt an das Beleuchtungssystem vermieden werden. Es ist aber auch möglich, dass Hülle als Schlichte ausgebildet sein kann, welche vorzugsweise Polyamid (PA), Polyimid (Pl) oder Polymethylmethacrylat (PMMA) oder Wachs, wachsähnliche Bestandteile oder Alkylsilan aufweist oder aus zumindest einem dieser Materialien gefertigt sein kann.

Der Diffusor-Grundkörper kann eine Matrix aus transparentem Kunststoff, insbesondere zur verbesserten Verarbeitung, Glas, Quarzglas oder transparenter Glaskeramik umfassen, oder aus dieser bestehen, wobei die Streuelemente bei einer
- Kunststoff-Matrix einen porösen oder pigmentierten oder eingefärbten Kunststoff umfassen oder aus diesem bestehen,
- bei einer Glas-Matrix Poren, Partikel, poröse oder pigmentierten oder eingefärbte oder Inhomogenitäten beinhaltende Glas- oder Glaskeramik, oder Glaskeramik-Elementen und die darin umfassten Kristallite umfassen oder aus diesen bestehen,
- bei einer Quarz-Matrix Poren, poröses Quarzglas oder keramische oder polykristalline Partikel umfassen oder aus diesen bestehen,
- bei einer transparenten Glaskeramik-Matrix Poren, Partikel, poröse oder pigmentierte oder eingefärbte oder Inhomogenitäten beinhaltendes Glas oder Glaskeramik, oder Glaskeramik-Elemente und den darin umfassten Kristalliten umfassen oder aus diesen bestehen, oder
- eine Kombination der jeweiligen Streuelemente
umfassen.

Die Inhomogenitäten des Glases oder der Glaskeramik, die die Streuelemente bei Glas- oder Glaskeramik-Matrixlösungen bilden, umfassen Phasenseparation, Entmischungen und/oder partikuläre Einlagerung, Keime und/ oder Kristallite, und die Streuwirkung ist gezielt mittels eines Temperatur-Zeit-Prozesses einstellbar. Hierbei soll die Konzentrationen der Streuelemente im Streubereich von 10 ppm bis 1000 ppm und bevorzugt von 20 ppm bis 100 ppm betragen. Hierbei bezieht sich die Konzentrationsangabe in ppm auf den Anteil der Streupartikel im Verhältnis zu den Masseanteilen der Bestandteile des jeweiligen Materials, insbesondere den Kunststoff, die Glas-Matrix oder die Quarz-Matrix, in welchem die Streupartikel eingelagert sind. Hierbei weisen die jeweils gebildeten Streuelemente, diese bedeutet beispielsweise die Poren, Partikel, porösen oder pigmentierte oder zum Beispiel weiß eingefärbten oder Inhomogenitäten beinhaltenden Glas- Oder Glaskeramik-Elemente und die darin umfassten Kristallite, bevorzugt einen Durchmesser von 10 nm bis 1000 nm auf, besonders bevorzugt von 100 nm bis 800 nm auf.

Vorteilhaft ist auch, wenn das Diffusor-Element Borosilikat-, Phosphat-Kron-Glas, Blei-Silikat-Glas, Zinn-Silikat- oder Alkali-Zink-Glas und/oder die Streuelemente Weißglas-Stäbchen enthalten, die insbesondere mit einem Hüllrohr, vorzugsweise aus Borosilikatglas umschlossen sind. Dieses hat den Vorteil, dass der Diffusor-Grundkörper als Ganzes im Röntgenbild zumindest teilweise oder abschnittsweise erkennbar ist und somit die Position des Diffusors im Körper eines Patienten bestimmbar ist. Im Hinblick auf Anwendungswellenlängen von 0,8 µm bis etwa 2,2 µm, zum Beispiel für die eingangs erwähnten EVLT-Anwendungen können auch spezielle IR-transparente Gläser, wie sie beispielsweise ein Phosphat-Kron-Glas, oder ein Blei-Silikat-Glas zum Einsatz kommen.

Bei einem Quarzglas-basierten Ansatz können insbesondere Anwendungen im UV und/ oder IR-Bereich bis etwa 2,5 µm Wellenlänge adressiert werden, wenn das Quarzglas besonders wenig OH-Gruppen aufweist. Als weiterer Vorteil ist hier die extrem hohe thermische Beständigkeit und die sehr niedrige Eigenabsorption von Quarz zu nennen, die insbesondere größere Laserleistungen bis 50 W in der Anwendung erlaubt. Neben Streuelementen aus porösem Quarzglas können auch Streuelemente aus oder mit keramischen Pigmenten, zum Beispiel Titandioxid, Zirkonoxid oder Aluminiumoxid zum Einsatz kommen. Quarzglas-basierte Diffusor-Grundkörper lassen sich besonders gut an aus Quarzfasern gebildete Lichtleiter spleißen, wobei die Quarzfasern aus einem Kern und einem Mantel, oder auch Cladding genannt, bestehen, die sich geringfügig in deren Brechzahl unterscheiden. Das Cladding kann auch aus organischen Materialien, wie beispielsweise Fluor-Kunststoff, PMMA oder Polyimid, bestehen.

Glaskeramik-basierte Ansätze für den Diffusor-Grundkörper und/oder die Streuelemente können aus einer transparenten Alumosilikat-Hochquarz- Mischkristall Glaskeramik gebildet sein. Diese ist extrem thermoschockbeständig und besitzt eine hohe spektrale Transmission bis etwa 2,5 µm. Als Streuelemente eignet sich beispielsweise eine Keatit-Glaskeramik, die durch einen geeigneten Temper-Prozess aus der Hochquarz-Mischkristall- Glaskeramik hergestellt werden kann. Weiterhin eignen sich Cordierit- glaskeramiken, oder Magnesium-Aluminium-Silikat Glaskeramik als Diffusor-Grundkörper und/oder Streuelemente. Ein hinsichtlich seines Herstellprozesses besonders bevorzugter Diffusor-Grundkörper ergibt sich, wenn der Diffusor-Grundkörper aus Lichtleitstäben aus Boro-Silikat-Glasstäben, Zinn-Silikat-Glasstäben, oder Alkali-Zink-Silikat-Glasstäben und/oder die Streuelemente aus Weißglas-Stäbchen gebildet sind, die vorzugsweise mit einem Hüllrohr aus Borosilikatglas, Zinn-Silikat-Glas, oder Alkali-Zink-Silikat-Glas umschlossen sind und die Vorform ausbilden.

In einer Weiterbildung der Erfindung können sowohl der Diffusor-Grundkörper und das Hüllrohr aus gleichartigem Glas bestehen. Der Brechwert des Hüllrohres ist dabei bevorzugt nicht größer als der des Glases der Matrix, insbesondere bevorzugt sind beide Brechwerte gleichgroß. Dies begünstigt die Auskopplung des im Diffusor gestreuten Lichtes. Damit sind kostengünstige Prozesse zur Herstellung von Diffusoren ermöglicht, die es erlauben diese in nahezu beliebiger Lange mit homogener Abstrahl- Intensität zu erhalten.

Die Erfindung betrifft auch ein Verfahren zum Herstellen von einem Beleuchtungssystem für ein medizintechnisches Therapie- und/ oder Diagnosesystem, insbesondere zur Anwendung an lebendem Gewebe, aufweisend wenigstens eine Lichtquelle, einen Lichtleiter, der an einem proximalen Ende an die Lichtquelle anschließbar oder dieser zuordenbar ist, und ein optisches Element, welches bevorzugt als Diffusor-Element ausgebildet ist und an einem distalen Ende des Lichtleiters angeordnet ist. Das Licht aus dem Lichtleiter ist in das optische Element einkoppelbar, und das Verfahren weist folgende Schritte auf:
- Bereitstellen des Diffusor-Elements mit einer Mantelfläche,
- zumindest abschnittsweise Bedecken der Mantelfläche mit einer Licht-reflektierenden Reflektorschicht, die vorzugsweise eine Spiegelschicht aufweist, sodass das optische Element einen Licht-reflektierenden Bereich aufweist, welcher von der Reflektorschicht bedeckt ist, und einen Licht-durchlässigen Bereich aufweist, welcher von der Reflektorschicht frei bleibt, sodass das vom optischen Element abzustrahlende Licht zumindest teilweise reflektierbar ist, und Licht gezielt am Licht-durchlässigen Bereich abstrahlbar. Die Reflektivität der Reflektorschicht ist für zumindest einen Wellenlängenbereich größer als 90%.
Idealerweise weist das mit der Reflektorschicht bedeckte Beleuchtungssystem zumindest eine, bevorzugt mehrere der zuvor beschriebenen Eigenschaften oder Merkmale auf, um die entsprechenden Vorteile zu erzeugen. Die Reflektorschicht erstreckt sich bevorzugt in axialer Richtung, insbesondere der Längsachse des optischen Elements. Die Mantelfläche kann auch als Mantelschicht oder Grundschichtbereich verstanden werden, dessen Wandstäre vorzugsweise im Bereich von 1 bis 100 µm liegt.

Vorteilhaft ist auch, wenn die Mantelfläche durch Erzeugen eines Grundschichtbereichs mit zumindest einer veränderten Oberflächeneigenschaft, insbesondere mittels chemischer oder physikalischer Prozesse zur Veränderung der Oberflächeneigenschaften der Mantelfläche, vorbehandelt oder aktiviert wird, sodass die Mantelfläche auch als Mantelschicht oder Grundschichtbereich verstanden werden kann, dessen Wandstäre vorzugsweise im Bereich von 1 bis 100 µm liegt. Die Reflektorschicht kann demnach unmittelbar auf der Mantelfläche, welche insbesondere nicht vorbehandelt ist, aufgebracht werden oder auf der Mantelschicht, beziehungsweise des Grundschichtbereichs.

Der Grundschichtbereich kann eine oberflächliche Schicht der Mantelfläche mit zumindest einer veränderten Oberflächeneigenschaft, insbesondere einer erhöhten Oberflächenenergie und/oder einer erhöhten Anzahl von Sauerstoffradikalen umfassen, welche eine gute Anhaftung folgender Beschichtungen, beispielsweise der Haftschicht und/oder der Reflektorschicht gewährleisten. Eine solche oberflächliche Teilschicht der Mantelschicht kann beispielsweise herstellbar oder hergestellt sein durch chemische oder physikalische Prozesse zur Veränderung der Oberflächeneigenschaften der Mantelfläche oder Mantelschicht, insbesondere Plasmabehandlung (z.B. Niederdruck-Plasma oder atmosphärisches Plasma), UV-Behandlung, Lichtbogenentladung (Corona) und/oder durch chemische Behandlung, z.B. mittels alkalischen Reinigern im Ultraschallbad, oder eine Kombination solcher Verfahren. Mittels Plasmen lassen sich außerdem Fette, Öle oder ähnlich Rückstände entfernen und zusätzlich Sauerstoffradikale aktivieren. Mit anderen Worten kann der Grundschichtbereich oder eine unterste Schicht des Grundschichtbereichs durch einen die äußere Oberfläche umfassenden radialen Teil der Mantelfläche gebildet sein. Der Grundschichtbereich kann also z.B. auch aus einer einzelnen Schicht bestehen, wobei diese einzelne Schicht mittels chemischer oder physikalischer Prozesse auf Basis der Mantelfläche des optischen Elements hinsichtlich der Oberflächeneigenschaften gebildet ist.

Der Grundschichtbereich kann aber auch aus einer Abfolge von Schichten bestehen, wobei z.B. die unterste Schicht des Grundschichtbereichs durch chemische oder physikalische Prozesse auf Basis der Mantelfläche des optischen Elements hinsichtlich der Oberflächeneigenschaften gebildet ist und auf dieser untersten Schicht weitere Schichten aufgebracht sind.

Es ist auch denkbar, dass die Reflektorschicht auf der Mantelfläche aufgebracht wird, insbesondere mittels Kathoden-Zerstäubung, Hochfrequenz-Zerstäubung, Reaktiv-Zerstäubung, Magnetron-Zerstäubung, Aufdampfen, insbesondere Ionenstrahl-Aufdampfen und/oder thermischen Aufdampfen. Das Erzeugen zumindest einer Schicht, bevorzugt mehrerer Schichten der Reflektorschicht kann neben den genannten Methoden insbesondere auch andere Beschichtungsverfahren, beispielsweise Vakuumverfahren (z.B. Ionenstrahl- oder thermisches Aufdampfen, chemische Gasphasenabscheidung (CVD, z.B. PECVD, insbesondere PICVD) betreffen. Als weitere Beschichtungsverfahren zum Aufbringen einer oder mehrerer Schichten der Reflektorschicht sind darüber hinaus Verfahren aus der flüssigen Phase, wie z.B. Tauchbeschichtung oder Sprühbeschichtungen möglich. Hierbei können weitere Funktionen wie Reibwertreduzierung mit funktionalisiert werden.

Dabei können die zur Beschichtung vorgesehen Substanzen, insbesondere Oxide, Nitrite oder Oxinitride von Si, Al, Ti, Zr, Hf, Y, Zn, beispielsweise mittels eines Sputterprozesses von einem sogenannten Sputtertarget appliziert werden. Es ist möglich, dass diese Materialien als metallische Targets oder als teilkeramische Targets vorliegen können. Die Reinheit der Targets wird typischerweise mit 99% oder mehr angegeben. Geringere Reinheiten sind aber auch möglich. Hierbei sind dann gegebenenfalls höhere Schichtdicken erforderlich.

Es kann vorgesehen sein, dass eine Haftschicht oder eine Haftvermittlerschicht auf der Mantelfläche und/oder eine Passivierungsschicht auf der Reflektorschicht aufgebracht wird, vorzugsweise mittels Kathoden-Zerstäubung, Hochfrequenz-Zerstäubung, Reaktiv-Zerstäubung, Magnetron-Zerstäubung, Aufdampfen, insbesondere Ionenstrahl-Aufdampfen und/oder thermischen Aufdampfen. Dabei kann der Grundschichtbereich als Haftvermittlerschicht ausgebildet werden. Demnach kann vorgesehen sein, dass zwischen dem Grundschichtbereich und der darauf aufgebrachten Reflektorschicht eine größere Haftung besteht, als sie zwischen einer behandelten oder unbehandelten Mantelfläche und einer auf dieser aufgebrachten Reflektorschicht bestehen würde. Mit anderen Worten kann eine erhöhte Haftung bestehen zwischen der Reflektorschicht und insbesondere der darunter befindlichen obersten Schicht des Grundschichtbereichs, welche ausgebildet sein kann als eine Schicht mit erhöhter Oberflächenenergie und/oder erhöhter Anzahl von Sauerstoffradikalen oder als eine auf der Mantelfläche durch Beschichtung aufgebrachten Haftschicht.

Es ist auch möglich, dass der Grundschichtbereich aus einer Mehrzahl von Haftschichten besteht, wobei zumindest eine oder eine jede oberhalb der untersten Haftschicht aufgebrachte Haftschicht eine höhere Haftung auf der darunter befindlichen Haftschicht aufweist als sie auf der Haftschicht, welche sich unter der darunter befindlichen Haftschicht befindet, aufweisen würde. Mit anderen Worten kann die Mantelfläche oder das optische Element mit einer einzelnen oder mit mehreren Haftschichten, welche den Grundschichtbereich bilden, beschichtet werden. Für zumindest eine Haftschicht ist der Einsatz zumindest eines Materials aus der Gruppe Si, Al, Ti, Zr, Hf, Y, oder Zn, vorgesehen, vorzugsweise als Oxid, Nitrit, oder Oxinitrid. Es können aber andere auch Substanzen zum Einsatz kommen, wie Borid, Carbid, oder Carbonitrid. Bevorzugte Schichtsysteme umfassen beispielsweise TiO₂.

Der Grundschichtbereich, beziehungsweise die Haftschicht und/oder die Passivierungsschicht kann eine amorphe Struktur, aber auch eine kristalline oder polykristalline Struktur aufweisen, insbesondere, wenn diese durch Beschichtung aufgebrachte Schicht/en aufweisen. Als typische Beispiele amorpher Beschichtungen sind SiO₂, Si₃N₄, Al₂O₃, AlSiOx oder BN zu nennen, als typische Beispiele für kristalline Beschichtungen sind anatases oder rutiles TiO₂, γ-Al₂O₃ oder kristallines AIN zu nennen. Insbesondere können aber auch Mischphasen aus amorph und kristallin ausgebildet werden.

Ein weiteres bevorzugtes Beleuchtungssystem sieht eine Spiegelschicht oder die Reflektorschicht vor, welche ein unterschiedliches Reflektionsverhalten bzw. Transmissionsverhalten für unterschiedliche Wellenlängen, insbesondere für nah beieinander liegenden Wellenlängen λ₁ und λ₂ aufweist, wobei der Wellenlängenunterschied Δλ = | λ₁-λ₂ | < 200 nm, bevorzugt < 100 nm betragen kann. Damit kann ein in seiner Abstrahlcharakteristik umschaltbares Beleuchtungssystem realisierbar werden, wobei über die Lichtquelle diese unterschiedlichen Wellenlängen λ₁ und λ₂ einstellbar sind. Der Grundgedanke ist dabei, die Reflektor- bzw.- Spiegelschicht derart auszulegen, dass bei der ersten Wellenlänge λ₁ die seitliche auf dem Mantel aufgebrachte Reflektionsschicht weitgehend transparent ist und durchstrahlt werden kann. Damit kann das Abstrahlverhalten eines rundum abstrahlenden Diffusors erreicht werden. Schaltet man auf die zweite Wellenlänge λ₂, wirkt nun die seitliche Reflektorschicht als Spiegel und es kommt zu einer seitlich in einem Sektor gerichtete Fokussierung der Abstrahlung. Für die zwei unterschiedliche Wellenlängen λ₁ und λ₂ weist die Spiegelschicht oder die Reflektorschicht insbesondere eine abweichende Reflektivität und/oder Transmission von vorzugsweise zumindest 10 %, besonders bevorzugt zumindest 30 %, auf. Damit kann insbesondere in vorteilhafter Weise erreicht werden, dass beim Umschalten der Wellenlänge eine signifikante Änderung der Abstrahlung zwischen vorwiegend rundum abstrahlend und fokussiert in einen Sektor abstrahlend ermöglicht wird. Die seitliche Reflektorschicht ist daher insbesondere eher schmalbandig hisichtlich der Wellenlänge ausgelegt. Vorteilhaft wäre dabei, wenn der distale Reflektor eine hohe Reflektion sowohl für λ₁ und λ₂ aufweist. Insbesondere ist dies bei medizintechnischen Anwendungen des Beleuchtungssystems von Vorteil. Beispiele für derart dicht beieinanderliegenden Wellenlängen λ₁ und λ₂ wären die Wellenlänge 980 nm und 1064 nm, welche oft bei medizintechnischen Anwendungen zum Einsatz kommen. Realisieren läßt sich so ein Verhalten insbesondere mit den zuvor beschriebenen dielektrischen Schichtsystemen. Hier sei angemerkt, dass natürlich λ₁ und λ₂ auch weiter (> 200 nm) auseinanderliegen können, was aber bei den medizintechnischen Anwendungen dazu führen kann, dass die Absorptionseigenschaften des Gewebes bei derart unterschiedlichen Wellenlängen sehr unterschiedlich sein können. Daher können die Wellenlängen nicht beliebig sein u./o. beliebig weit auseinanderliegen.

Damit einhergehend kann eine weitere Anwendung genannt werden, bei welcher mit unterschiedlichen Wellenlängen, welche insbesondere umschaltbar sein können, unterschiedlich weite Penetration der Strahlung erzielt werden kann, was insbesondere im Hinblick auf das Eindringverhalten in Gewebe aus chirurgischer Sicht einen Vorteil bieten kann.

Das Beschichten des optischen Elements mit der Reflektorschicht umfasst zunächst das Erzeugen eines Grundschichtbereichs bestehend aus einer einzelnen Haftschicht und/oder einer Aktivierung der Mantelfläche oder einer Abfolge von Haftschichten und danach das Aufbringen (auf dem Grundschichtbereich) einer Spiegelschicht bestehend aus einer einzelnen Schicht oder einer Abfolge von, insbesondere im Wechsel hoch und niedrig brechenden Schichten, beziehungsweise eines Schichtsystems aus hoch und niedrig brechenden Schichten. Eine Aktivierung der Mantelfläche kann verstanden werden als ein Entfernen von Rückständen und/oder das Erzeugen einer veränderten Oberflächeneigenschaft, insbesondere einer erhöhten Oberflächenenergie und/oder einer erhöhten Anzahl von Sauerstoffradikalen.

Das Beschichten oder Aktivieren erfolgt vorzugsweise bei Temperaturen von unter 50°C. Diese relativ niedrige Temperatur hat insbesondere den Vorteil, dass optische Elemente mit einer Polymerschicht als Hülle beschichtet werden können. Bevorzugt ist auch eine Prozessierung im Vakuum und insbesondere ohne Vakuumbruch. Es kann demnach beispielsweise im Vakuum zuerst der Grundschichtbereich erzeugt werden und danach in demselben Vakuum die Spiegelschicht aufgebracht werden. Ferner kann danach in demselben Vakuum noch die Passivierungsschicht aufgebracht werden.

Eine bevorzugte Verwendung des Beleuchtungssystems, wie es zuvor in seinen verschiedenen Ausführungsvarianten beschrieben wurde, sieht den Einsatz für eine photodynamische Therapie (PDT) oder Photoimmuno-Therapie (PIT) beispielsweise zur Tumortherapie, für eine endovenöse Lasertherapie (EVLT) beispielsweise zur Behandlung von Krampfadern, für eine Laser-induzierte interstitielle Thermotherapie (LITT) oder für Anwendungen im Bereich der Dentalmedizin, Augenheilkunde sowie Dermatologie vor, wie diese eingangs beschrieben wurden. Im Bereich Dentalmedizin sind hier insbesondere Applikationen zur Wund- oder Parodontose-Behandlung zu nennen. Darüber hinaus gibt es Anwendungen in der Hirnforschung, bei denen mittels Licht einzelne Gehirnbereiche stimuliert und damit Krankheitssymptome behandelt werden können.

Eine weitere Verwendung des Beleuchtungssystems, wie es zuvor in seinen verschiedenen Ausführungsvarianten beschrieben wurde, sieht den Einsatz für eine photodynamische Therapie (PDT) oder Photo-Immuno-Therapie (PIT) zur Tumortherapie vor, wobei zumindest ein Lichtleiter mit dem Diffusor-Element aus anderen Diffusor-Elementen abgestrahltes Licht aufnimmt und über den Lichtleiter einem Detektor zur spektroskopischen Analyse weiterleitet. Dabei werden dem Patienten neben den verschiedenen Lichtaussendenden Diffusor-Lichtleitern auch lichtempfangende Diffusor-Lichtleiter appliziert, wobei anhand der spektralen Unterschiede zwischen eingekoppeltem und empfangenen Licht auf ein Ansprechen der PDT-Behandlung geschlossen werden kann (siehe dazu Finlay et al., Proc. SPIE Int. Soc. Opt. Eng. 2014, June 14; 5315: Page 132-142). Weiterhin können derartige Systeme auch zur Dosimetrie bei z.B. PDT- oder PIT-Behandlungen verwendet werden.

Darüber hinaus sind auch Anwendungen im industriellen Bereich vorteilhaft, etwa als Komponente einer Vorrichtung für industrielle Anwendungen zur gezielten Ausleuchtung von Hohlräumen, beispielsweise zur Inspektion von schwer zugänglichen Stellen beispielsweise an oder in einer Maschine, bei denen es insbesondere auf eine homogene Ausleuchtung ankommt, Durchstrahlen von Werkstücken mit kleinen Öffnungen oder auch spektroskopische Anwendungen oder in der Biochemie, bei der durch Licht biochemische In-Vitro-Reaktionen stimuliert werden, also zur Bestrahlung von Proben im Rahmen der In-Vitro-Diagnostik. Auch in der gezielten Aushärtung von Harzen oder Klebern oder diese umfassende Werkstoffe, ebenso dort wo eine homogene Ausleuchtung notwendig ist oder Fügestellen schwer zugänglich sind, ist eine Anwendung zu sehen.

Die Erfindung wird nachfolgend genauer anhand der beigeschlossenen Figuren erläutert. In den Figuren bezeichnen gleiche Bezugszeichen jeweils gleiche oder entsprechende Elemente. Es zeigen:
- Fig. 1: Schematische Darstellung eines Beleuchtungssystems mit einem Lichtleiter und einem in eine Richtung sektional abstrahlenden Diffusor-Elements in einer PDT- oder PIT-Anwendung;
- Fig. 2: Schematischen Darstellung eines Diffusor-Elements;
- Fig. 3: Vergleich der unterschiedlich beschichteten Oberflächen nach einem Abriebtest;
- Fig. 4: Schematische Darstellung eines Ausführungsbeispiels einer Reflektorschicht;
- Fig. 5: Schematischer Darstellung eines Ausführungsbeispiels mit einer Reflektorschicht, einer Haftschicht und einer Passivierungsschicht;
- Fig. 6: Schematischer Darstellung eines Ausführungsbeispiels mit einer dielektrischen Reflektorschicht, einer Haftschicht und einer Passivierungsschicht;
- Fig. 7: Verlaufsdiagramm der Reflektivität in Abhängigkeit von der Wellenlänge;
- Fig. 8: Verlaufsdiagramm der Reflektivität in Abhängigkeit von der Wellenlänge für verschiedene Licht-Einfallswinkel;
- Fig. 9: Verlaufsdiagramm der Reflektivität in Abhängigkeit von der Wellenlänge und dem Einfallswinkel für einen Interferenzreflektor.

Bei der nachfolgenden Beschreibung der detaillierten Ausführungsformen bezeichnen gleiche Bezugszeichen in den beiliegenden Figuren jeweils gleiche oder gleich wirkende Bestandteile. Zum besseren Verständnis werden die nachfolgenden Definitionen vorgenommen. Im Sinne der vorliegenden Offenbarung umfasst der Begriff des Beleuchtungssystems Beleuchtungsvorrichtungen und insbesondere Beleuchtungsvorrichtungen, welche zur Anwendung für medizintechnische Zwecke geeignet und insbesondere, soweit diese mit lebendem Gewebe in Kontakt treten sollen, zumindest abschnittsweise desinfizierbar und/ oder sterilisierbar sind. Die Bezeichnung "für ein medizintechnisches Therapie- und/oder Diagnosesystem" umfasst ohne Beschränkung hierauf insbesondere auch die Eignung, Verwendung oder Anwendung des hier offenbarten Beleuchtungssystems selbst als medizinisches Therapie- und/oder Diagnosesystem.

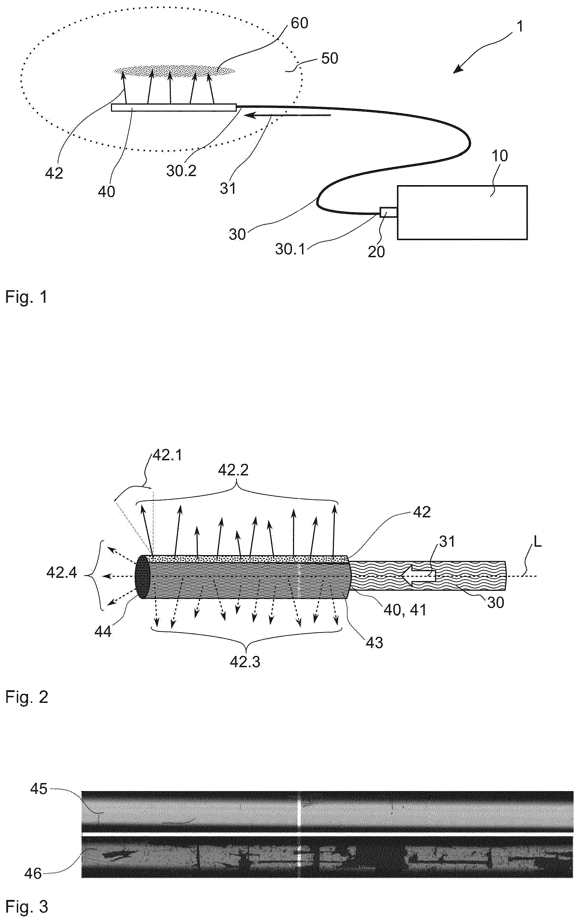

Fig. 1 zeigt schematisch den Aufbau eines erfindungsgemäßen Beleuchtungssystems 1. Beispielhaft ist eine medizintechnische PDT-Anwendung dargestellt. Im gezeigten Beispiel umfasst das Beleuchtungssystem 1 eine LED- oder Laser-Lichtquelle 10, welche im Betriebszustand Licht in einem bestimmten Spektralbereich aussendet. Für PDT- bzw. PIT-Anwendungen, wie sie eingangs beschrieben sind, werden Laser verwendet, die auf einen zuvor verabreichten biochemisch modifizierten Farbstoff (Photosensitizer) abgestimmte Wellenlänge üblicherweise im sichtbaren Bereich, beispielsweise im grünen Spektralbereich bei 532 nm oder im roten Spektralbereich bei zum Beispiel 690 nm, aussenden.

Ein Lichtleiter 30 ist mit einem Stecker 20 oder anderem Anschlusselement, beziehungsweise Verbindungselement an seinem proximalen Ende 30.1 an die Laser-Lichtquelle 10 angeschlossen. Als proximales Ende 30.1 wird hierbei das Ende des Lichtleiters 30 bezeichnet, in welches Licht eingekoppelt wird. Am distalen Ende 30.2 weist der Lichtleiter 30 ein optisches Element in Form eines Diffusor-Elements 40 auf, welches in oder in die Nähe eines Tumor-Gewebe 60 eingebracht wird, welches sich innerhalb eines gesunden Gewebes 50 gebildet hat und daher an dieses angrenzt. Als distales Ende 30.2 wird hierbei das andere Ende des Lichtleiters 30 bezeichnet, welches in der Regel zu dem proximalen Ende 30.1 des Lichtleiters 30 entfernt angeordnet ist und aus welchem insbesondere Licht austritt.

Die Laserstrahlung gelangt dabei über eine Lichteinkopplung 31 über den Lichtleiter 30 am Diffusor-Element 40 in das Diffusor-Element 40, welches beispielsweise aus einem Diffusor-Grundkörper 41 gebildet ist. Das Licht wird über die Länge L des Diffusor-Elements, welche durch die Längsachse L gekennzeichnet ist, seitlich abgestrahlt, beziehungsweise als Lichtauskopplung 42 im lichtdurchlässigen Bereich ausgekoppelt. Dabei kommt es auf eine möglichst homogene Abstrahlung über die Länge des Diffusor-Elementes 40 an. Insbesondere sind Intensitätsspitzen zu vermeiden. Im gezeigten Beispiel strahlt das Diffuser-Element 40 nur in eine Richtung, also sektional ins Tumorgewebe 60 ein. Durch eine photoinduzierte biochemische Reaktion, wie sie eingangs beschrieben ist, kommt es nach der Behandlung idealerweise zu einem Absterben des Tumor-Gewebes 60.

Ohne Beschränkung auf das hier gezeigte Beispiel werden in der Regel als Lichtleiter 30 Quarz-Fasern verwendet, wobei die Stecker 20 in der Regel als koaxialer Steckverbinder, sogenannte SMA-Stecker, ausgebildet sind, bei denen die Fasern vorzugsweise in den Stecker 20 geklebt sind. Vorteilhaft hinsichtlich der thermischen Belastbarkeit können auch Stecker 20 mit Neusilber-Hülsen sein, bei denen der Lichtleiter 30 in die Neusilber-Hülse formschlüssig, insbesondere durch plastische Verformung eingebracht/ gecrimpt, ist. Darüber hinaus können bei größeren Laser-Leistungen auch Stecker 20 zum Einsatz kommen, bei denen das Faserende des Lichtleiters 30 durch ein Kegelprisma geschützt ist, was vorteilhaft bei Fehljustagen sein kann.

Fig. 2 zeigt schematisch und beispielhaft den Aufbau des Diffusor-Elementes 40. Das Diffusor-Element 40 besteht aus einem Diffusor-Grundkörper 41, welcher bevorzugt an den Lichtleiter 30 gespleißt oder angeklebt ist. Der Lichtleiter 30 besteht bei den zuvor beschriebenen Anwendungen meist aus einer Quarzfaser mit einem Kern. Dabei hat der Kern einen Brechungsindex n1 und einen Kerndurchmesser von üblicherweise zwischen 100 µm und 1000 µm, bevorzugt zwischen 200 µm und 600 µm, sowie einen Mantel oder eine Mantelfläche mit einem Brechungsindex n2, wobei gilt n1 > n2. Die damit üblicherweise erzielbare numerische Apertur NA beträgt etwa 0,22 oder weniger, beispielsweise 0,1. Durch spezielle Dotierungen können auch höhere NA-Werte, bis zu 0,4 oder 0,6 erzielt werden. Die Lichteinkopplung 31 aus dem Lichtleiter 30 erfolgt über eine entsprechende Einkoppelfläche des Diffusor-Grundkörpers 41.

Weiterhin kann es bei einer gespleißten Verbindung vorteilhaft sein, wenn die Verbindung zwischen Diffusor-Grundkörper 41 und Lichtleiter 30 zweistufig ausgeführt ist. Zunächst wird nur ein kurzes Stück (typ. wenige 10 mm lang, z.B. ca. 10 bis 20 mm) des Lichtleiters 30 an den Diffusor-Grundkörper 41 an gespleißt, dieser dann beschichtet und danach das eigentliche Pigtail, bestehend aus Lichtleiter 30 und Stecker 20, an das kurze Stück des Lichtleiters 30 angespleißt. Dies ist insbesondere von Vorteil, dass durch den Spleiß-Prozeß, bei dem lokal eine hohe Energiedichte, induziert durch Laser und/oder Corona-Entladungen, die Reflektor-Beschichtung nicht thermisch zerstört wird bzw. die Reflektionseigenschaften unzulässig verändert werden. Zudem kann beim Beschichtungsprozeß der Diffusor-Grundkörper 41 mit dem nur kurzen Abschnitt des Lichtleiters 30 in der Beschichtungskammer einfacher eingebaut werden. Der Handlings-Aufwand kann damit deutlich gegenüber einem Handling mit kompletten Pigtail reduziert werden.

Der Diffusor-Grundkörper 41 oder das optische Element umfasst beispielsweise eine Matrix aus Matrix-Elementen mit eingelagerten Streuelementen und/oder ein Hüllrohr. Der Diffusor-Grundkörper 41 kann aber auch aus einem Glas bestehen, dessen Streueigenschaften durch eine thermische Behandlung mit definierter Temperatur-Zeit-Steuerung gezielt eingestellt werden kann. Denkbar für den Diffusor-Grundkörper 41 ist auch eine Matrix aus einer Glaskeramik, bei der ebenfalls durch eine gezielte Temperatur-Zeit-Behandlung die Kristallit-Dichte und/oder die Größe der Kristallite gezielt einstellbar sind und somit auch die Streueigenschaft beeinflußbar ist. Ebenso denkbar sind auch Kunststoff-basierte optische Elemente, bei denen als Lichtleiter 30 auch eine Polymer-Optische Faser (POF) angebracht ist.

Zur Vermeidung von Streulicht aus der Verbindungszone zwischen Lichtleiter 30 und Diffusor-Grundkörper 41 aber auch als mechanische Stabilisierung dieser Verbindungszone kann eine Hülse aus Kunststoff, Glas, Metall oder keramischen Werkstoff vorgesehen sein.

Das Diffusor-Element 40 weist eine Reflektorschicht 43 sowie optional auch einen Reflektor, beziehungsweise eine weitere Reflektorschicht am distalen Ende 44 auf. Damit ergeben sich als Lichtauskopplung 42 im Wesentlichen 3 Beiträge: Eine in einem vorgebbaren Abstrahlwinkel 42.1 seitliche Lichtauskopplung 42.2, eine rückwärtige Lichtauskopplung 42.3 durch die Reflektorschicht hindurch und eine distale Lichtauskopplung 42.4.

Für eine medizintechnische Anwendung, bei der gezielt gerichtet Gewebe bestrahlt werden soll, soll daher der Anteil der rückwärtigen Lichtauskopplung 42.3 so gering wie möglich sein, was eine möglichst hohe Reflektivität der Reflektorschicht 43 erforderlich macht. Zudem kann es auch erforderlich sein, dass am distalen Ende 44 die distale Lichtauskopplung 42.4. weitgehend verhindert wird, was ebenfalls auch hier mit einer möglichst hohen Reflektivität der Reflektorschicht am distalen Ende 44 einhergeht. Zudem kann durch eine möglichst hohe Reflektivität die Rückreflektion am distalen Ende 44 und/oder die Effizienz der seitlichen Lichtauskopplung 42.2 gesteigert werden. In beiden Fällen sind die Reflektivitätswerte von über 90 %, bevorzugt über 95 % und besonders bevorzugt über 99 % gefordert. Zudem darf die Reflektorschicht auch keine große Absorption aufweisen, um eine zu starke Erwärmung zu verhindern.

Wichtig ist auch die Korrosionsbeständigkeit, insbesondere im Hinblick auf aufwendige Reinigungsprozesse und mechanischen Belastungen, beispielsweise, wenn das Das Diffusor-Element 40 mehrfach in einem Katheter verschoben wird und aufgrund dessen Reibungen entstehen. Die aufgebrachten Schichten der Reflektorschicht lassen sich daher Reinigungsprozessen unterziehen (z.B. mit Ethanol) oder bestehen sogar Abrasivtests, beispielsweise den Radiergummitest oder auch den "Tesa-Test" oder Taper-Test- .

Figur 3 zeigt daher einen Vergleich nach einem Radiergummitest: Oben im Bild dargestellt ist die als ein dielektrisches Mehrschichtsystem 45 im 4-Schicht-Design gestaltete Reflektorschicht 43. Im Bild unten ist zum Vergleich die Oberfläche bei einer metallischen Cr + Au-Reflektorbeschichtung 46. Zu erkennen ist, dass eine reine metallische Reflektorbeschichtung den Belastungen nicht ausreichend standhält und ein dielektrisches Mehrschichtsystem 45 bezüglich der mechanischen Belastbarkeit besser abgestimmt werden kann.

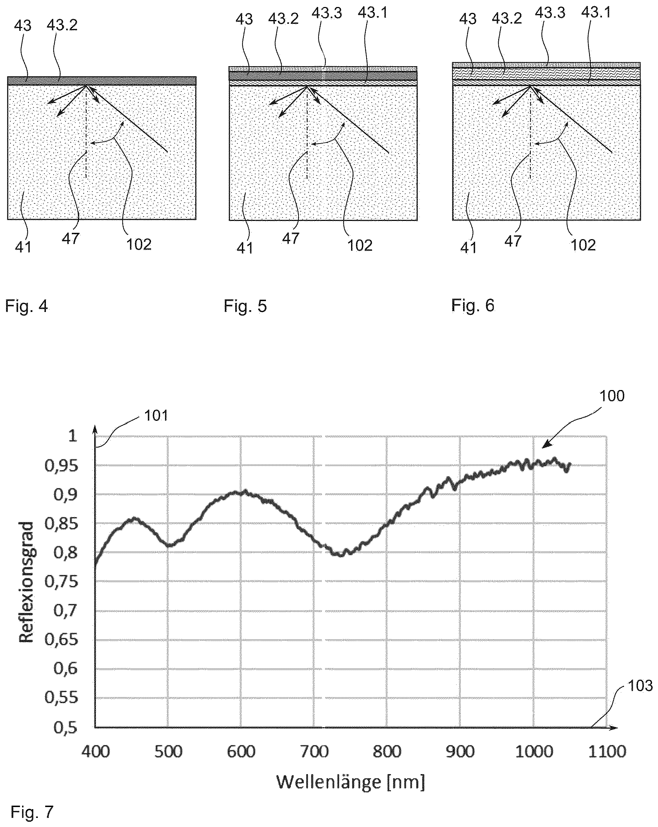

Die Figuren 4 bis 6 zeigen schematisch, nicht maßstabsgetreu dargestellte typische Ausführungsbeispiele der Reflektorschicht 43. Figur 4 zeigt einen einfachen Schichtaufbau für den Mantel-Reflektor 43, bei dem auf dem Diffusor-Grundkörper 41 eine metallische Spiegelschicht 43.2 abgeschieden wurde. Das zu reflektierende Licht kann dabei in unterschiedlichen Einfallswinkeln 102 zur Senkrechten 47 auf die Spiegelschicht 43.2, die Haftvermittlerschicht 43.1 oder die Reflektorschicht 43 treffen.

Vorteilhafterweise ist die maximale Reflektivität bei schräg zur Reflektorfläche einfallendem Licht größer als 50 %, bevorzugt größer als 70% und besonders bevorzugt größer als 90 %. Im Rahmen der vorliegenden Offenbarung wird schräg einfallendes Licht derart verstanden, dass das Licht in einem Winkel zwischen absolut 0° und bis zu 90°, oder vorzugsweise in einem Winkel größer als 50°, bevorzugt größer als 70° zur Senkrechten 47 der Reflektorschicht 43 auf die Reflektorschicht 43 trifft. Da das Licht im Wesentlichen längs des Diffusor-Elements 41 geleitet und gestreut wird, wird ein gewisser Anteil des Lichts schräg auf die Reflektorschicht 43 auftreffen. Es ist demnach vorteilhaft, wenn auch dieser Teil des Lichts reflektiert wird.

Metallische Spiegelschichten 43.2 können vorzugsweise aus Edelmetallen, wie Au, Ag, Pd oder Pt bestehen, welche eine hohe Reflektivität im sichtbaren Bereich des Lichtes aufweisen. Denkbar sind auch Legierungen aus mehreren Metallen oder mehrschichtig aufgebaute Metallschichten. Solche einfach aufgebauten Schichten können, wie in Figur 3 dargestellt, in manchen Fällen jedoch nicht praxistauglich im Hinblick auf mechanischen Abrieb, Haftung oder chemischen Angriff sein.

Figur 5 zeigt daher einen komplexeren, mehrschichtigen Aufbau mit einer Haftschicht 43.1 beziehungsweise Haftvermittlerschicht direkt auf dem Diffuser-Grundkörper 41, der eigentlichen Spiegelschicht 43.2 und einer zusätzlichen Passivierungsschicht 43.3. Hierbei ist zu beachten, dass die klassischen Haftvermittler für Edelmetalle wie Cr oder Ni durch die Lichtführung über die Faser, beziehungsweise das optische Element zu einer Reduktion der Reflexion führt. Selbst bei sehr geringen Schichtdicken der Haftschicht 43.1 von beispielsweise 10 nm ist dies der Fall. Als alternative Haftschichten 43.1 können dielektrische Schichten verwendet werden, um die Reflexion nicht signifikant zu reduzieren. Hierbei können Oxide, Nitride oder Oxinitride von Si, Al, Si, Al, Ti, Zr, Hf, und gegebenenfalls auch Y und Zn verwendet werden. Die Schichtdicken der als Spiegelschicht 43.2 ausgeführten Edelmetallschichten können zwischen 10 nm und 5000 nm liegen, vorzugsweise im Bereich zwischen 10 nm und 300 nm. Die Dicke der Haftschicht 43.1 oder Haftvermittlerschicht beträgt typischerweise maximal wenige 10 nm, bevorzugt im Bereich von 5 nm bis 50 nm.

Ein weiteres Beispiel für eine mögliche Spiegelschicht 43.2 ist eine Nicht-Edelmetallschicht mit hoher Reflexion wie beispielsweise Mg, Al oder Cu in ein optisches Design so eingebettet, dass sich die Reflexion erhöht. In der einfachsten Form wird ein 3-Schicht-Design aus TiO₂ (Mg), SiO₂ und Mg hergestellt, wobei die Reflexion durch die Verwendung einer hochbrechenden ersten Schicht, einer niedrigbrechenden zweiten Schicht und einer hochreflektierenden Metallschicht zu einer Erhöhung der Reflexion der Metallschicht führen kann. Durch eine weitere Schicht, insbesondere einer Passivierungsschicht 43.3 im Anschluss an die Metallschicht wird ein zusätzlicher Schutz gegenüber Korrosion gewährleistet. Diese zusätzlich Barriereschicht/Passivierungsschicht kann Oxide, Nitride oder Oxinitride von Si, AlSi, Al, Ti, Zr, Hf und ggf. auch Y und Zn enthalten. An dieser Position sind die optischen Daten der Schicht nicht relevant, weshalb auch Opfermetallschichten verwendet werden könnten, welche anstatt der Funktionsmetallschicht korrodieren. Dies können beispielsweise Al, Cu, Cr oder Ni sein. Aber auch andere Metalle sind denkbar.

Beispielhaft und ohne Beschränkung auf die vorgestellten Werte sind im Folgenden Beispiel 1 die Schichtdicken eines funktionalen 3 Schichters einer Reflektorschicht 43 dargestellt, welche in folgenden Bereichen liegen:
I. TiO₂ (als Haftschicht 43.1): Bereich: 30 nm bis 5000 nm, bevorzugt zwischen 30 nm und 300 nm, typischerweise 100 - 200 nm.
II. SiO₂: Bereich: 40 nm bis 5000 nm, bevorzugt zwischen 40 nm und 380 nm, typischerweise 150 bis 250 nm.
III. Mg: Bereich: 20 nm bis 2000 nm, bevorzugt zwischen 20 nm und 200 nm, typischerweise 50 bis 150nm.
IV. SiO₂: Bereich: 5 nm bis 5000 nm, bevorzugt zwischen 5 nm und 500 nm, typischerweise 100 bis 250nm.

Durch das dargestellte Design kann die Reflexion in einem Wellenlängenbereich von über 1000 nm auf über 90%, bevorzugt über 92% oder auch vorzugsweise über 95% erhöht werden, insbesondere bei einem Lichteinfall senkrecht zur Reflektorschicht 43. Darüber hinaus zeichnet sich das Schichtsystem dadurch aus, dass hohe Reflexionen in einem Winkelbereich von 0 bis +/-80°, gemessen zur Senkrechten der Reflektorschicht 43, erhalten bleiben, was bei der anvisierten Applikation im medizinischen Bereich, insbesondere bei der Bestrahlung von Gewebe notwendig ist. Hohe Reflexionen werden im Sinne der Erfindung verstanden als Reflexionen, welche in einem zuvor angegebenen Winkelbereich um die maximale Reflektivität bei senkrechtem Lichteinfall, größer sind als 50%, bevorzugt größer als 70%, bevorzugt größer als 90%.

Ein wesentlicher Vorteil einer Mg-basierten Schicht des Beispiels 1, ist die hohe Reflektivität als Nicht-Edelmetallbeschichtung, ähnlich wie bei Cu oder Al. Ein großer Vorteil dieser Variante ist die einfache Herstellbarkeit. Da nur wenige Schichten notwendig sind, ist auch eine Herstellung in großen Anlagen/ Inline-Anlagen möglich. Weiterhin ergeben sich in diesem Fall keine großen Anforderungen an die Uniformität. Zudem sind TiO₂- und SiO₂-Schichten als Standardprozesse gut beherrschbar. Ebenso ist eine Mg-Schicht als metallische Schicht einfach herzustellen. Ein weiterer Vorteil gegenüber weicheren Edelmetallschichten ist die gute Haftung und mechanische Beständigkeit. Die aufgebrachten Schichten lassen sich Reinigungsprozessen unterziehen (z.B. mit Ethanol) oder bestehen sogar, wie in Figur 3 gezeigt, Abrasivtests.

In Figur 6 ist exemplarisch analog zu den Figuren 4 und 5 ein weiteres Beispiel in Form eines interferenzoptischen dielektrischen Systems dargestellt. Hierbei setzt sich die Spiegelschicht 43.2 abwechselnd aus hoch- und niedrig-brechenden Schichten zusammen, so dass gemäß der Fresnelschen Beziehungen aus transparenten, dielektrischen Schichten hohe Reflexionen erzielt werden können. Dies erreicht man durch das Einstellen der Lambdaviertel-Schichtdicke für eine Referenzwellenlänge. Als Materialien für diesen Ansatz kommen als hochbrechende Schichten TiO₂, Ta₂O₅, Nb₂O₅ oder ZrO₂ in Frage oder allgemein Schichten mit einer Brechzahl von über 2,2. Die genannten Materialien können durch Dotierungen noch stabilisiert werden, wie z.B. durch Al, Si, Y, Zn. Als niedrigbrechende Schicht kommt insbesondere SiO₂ oder dotierte SiO₂-Varianten in Frage. Auch Oxide, Nitride, Fluoride (z.B. MgF₂) oder Oxinitride anderer Metalle sind möglich. SiO₂ mit einer geringer N₂-Dotierung ist ebenfalls denkbar und erfüllt die Anforderung einer niedrigen Brechzahl von vorzugsweise unter 1,5 sehr gut. Mit solchen Designs, wie beispielsweise eines hoch-niedrigbrechenden Schichtsystems aus 11 Schichten können breitbandige und hochreflektierende Reflektorschichten 43 oder Spiegelschichten 43.2 erzielt werden, wobei die Schichtdicken für TiO₂ beispielsweise bei 120 nm bis 130 nm und für SiO₂ beispielsweise bei 210 nm bis 220 nm, insbesondere für einen Wellenlängenbereich zwischen 1000 nm und 1100 nm liegen. Durch die Breitbandigkeit sind ebenfalls verschiedene Einfallswinkel in einem weiten Bereich bei definierter Referenzwellenlänge (hierbeispielsweise für 1064 nm) reflektierbar.

Figur 7 zeigt in einem Verlaufsdiagramm 100 die Reflektivität 101, beziehungsweise den Reflexionsgrad in Abhängigkeit von der Wellenlänge 103 für ein in Beispiel 1 beschriebenes Mehrschichtsystem aus TiO₂ / SiO₂ / Mg / SiO₂. Bei einem derartigen System ist der Reflexionsgrad bei Wellenlängen um die 600 nm, sowie im Wellenlängenbereich von etwa 900 nm bis etwa 1100 nm besonders hoch, beispielsweise über 90%.

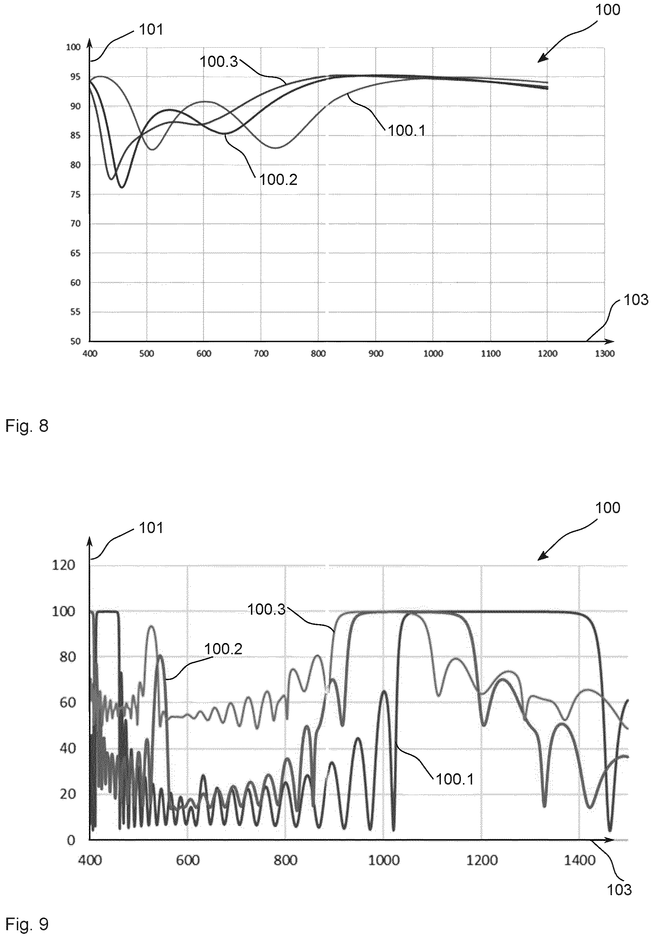

Figur 8 zeigt in einem weiteren Verlaufsdiagramm 100 die Reflektivität 101, beziehungsweise den Reflexionsgrad für das in Figur 7 dargestellte Schichtsystem in Abhängigkeit von der Wellenlänge 103 und für verschiedene Einfallswinkel 102. Der 1. Verlauf 100.1 zeigt einen Verlauf für einen 0° Einfallswinkel zur Senkrechten. Der 2. Verlauf 100.2 den für einen 60° Einfallswinkel zur Senkrechten und der 3. Verlauf 100.3 den für 80° Einfallswinkel zur Senkrechten gemessen. Hier ist zu erkennen, dass beispielsweise in einem Bereich von 1000 nm bis 1100 nm ein nahezu winkelunabhängiger Reflexionswert von 95% erzielt werden kann. Im gezeigten Beispiel wurde ein solches Schichtsystem für eine Anwendungswellenlänge von 1064 nm optimiert. Durch Variation der einzelnen Schichtdicken der Schichten der Spiegelschicht 34.2 kann eine derart hohe, nahezu winkelunabhängige Beschichtung für einen weiten Wellenlängenlängenbereich eingestellt werden.

Figur 9 zeigt in einem weiteren Verlaufsdiagramm 100 für ein insbesondere 11-schichtiges hoch-niedrig-brechendes Schichtdesign, aus TiO₂/ SiO₂ in verschiedenen Verläufen 100.1, 100.2, 100.3 die Reflektivität 101, hier normiert auf 100% für den Maximalwert, für unterschiedliche Einfallswinkel 102. Der 1. Verlauf 100.1 repräsentiert den 0° Einfallswinkel 102, der 2. Verlauf 100.2 repräsentiert den 60° Einfallswinkel 102 und der 3. Verlauf 100.3 repräsentiert den 80° Einfallswinkel 102 gegenüber der Senkrechten zur Spiegelschicht 43.2. Die Center-Wellenlänge beträgt hier ca. 1040 nm bis 1060 nm.
Mit solchen Schichtvarianten lassen sich sehr hoch reflektierende Designs erzielen, abhängig von der Absorption, beziehungsweise Trübung der verwendeten Schichten. Die Absorption ist abhängig von den verwendeten Materialien und dem Herstellungsprozess, die Trübung kann seine Ursache in Sauberkeit der Substrate, Defekte im Beschichtungsprozess, in einer Plasmapolymerisierung, in Rissbildung, oder Ähnlichem haben. Da ein Beschichtungsprozess sehr reproduzierbar und stabil sein muss, werden PVD basierte Prozesse wie Aufdampfen oder Sputtern an, beispielsweise Magnetron-Sputtern oder Ionenstrahl-Sputtern bevorzugt zur Herstellung der Reflektorschicht 43, der Haftschicht 43.1 und/oder der Passivierungsschicht 43.3 eingesetzt.
Der Beschichtungsprozess lässt sich beispielsweise über einen Magnetron-Sputterprozess darstellen. Hierbei umfasst der Prozess bei einem 3- oder 4- Schicht-System die Schritte:
- Reinigung der Substratoberfläche, beziehungsweise der Mantelfläche oder der Oberfläche des optischen Elements über einen Ultraschall-Reinigungsprozess,
- thermische Behandlung der Mantelfläche oder der Oberfläche des optischen Elements im Vakuum zur Verbesserung der Haftung durch Entwässerung der Oberfläche,
- reaktives Magnetron-Sputtern vom metallischen Target der TiO₂- und SiO₂-Schichten oder Sputtern vom keramischen Target bei TiO₂ beziehungsweise metallisches Sputtern von Mg,
- defektfreies Sputtern durch beispielsweise vertikale Anlagen- und Substratanordnung, beziehungsweise Anordnung des optischen Elements.
In einem weiteren Schritt, insbesondere einem zusätzlichen Prozessschritt kann eine Sauerstoffplasma-Vorbehandlung im Vakuum erfolgen. Dies dient der Verbesserung der Haftung durch Vorkonditionierung der Oberfläche. Es sind aber auch Plasmavorbehandlungen, insbesondere unter Atmosphärendruck vorteilhaft.
Bei metallische Schichten als Reflektorschicht ist es auch denkbar, dass diese zunächst auf einer Pre-Form des Diffusor-Rohlings aufgebracht werden und dann in einem Ziehprozess der Diffusor ausgezogen wird.

### Bezugszeichenliste

- 1: Beleuchtungssystem
- 10: Laser-Lichtquelle
- 20: Stecker
- 30: Lichtleiter
- 30.1: Proximales Ende des Lichtleiters
- 30.2: Distales Ende des Lichtleiters
- 31: Lichteinkopplung
- 40: Diffusor-Element
- 41: Diffusor-Grundkörper
- 42: Lichtauskopplung im lichtdurchlässigen Bereich
- 42.1: Abstrahlwinkel
- 42.2: Seitliche Lichtauskopplung
- 42.3: Rückwärtige Lichtauskopplung
- 42.4: Distale Lichtauskopplung
- 43: Reflektorschicht (Lichtreflektierender Bereich)
- 43.1: Haftschicht
- 43.2: Spiegelschicht
- 43.3: Passivierungsschicht
- 44: Reflektor distales Ende
- 45: Dielektrisches Mehrschichtsystem
- 46: Metallische Reflektorbeschichtung
- 47: Senkrechte
- 50: Gewebe
- 60: Tumorgewebe
- 100: Verlaufsdiagramm
- 100.1: 1. Verlauf
- 100.2: 2. Verlauf
- 100.3: 3. Verlauf
- 101: Reflektivität
- 102: Einfallswinkel
- 103: Wellenlänge
- L: Längsachse

## Patentansprüche

1. Beleuchtungssystem (1), insbesondere für ein medizintechnisches Therapie- und/oder Diagnosesystem, aufweisend wenigstens eine Lichtquelle (10), einen Lichtleiter (30), der an einem proximalen Ende an die Lichtquelle (10) anschließbar oder dieser zuordenbar ist, und ein optisches Element, welches bevorzugt als Diffusor-Element (40) ausgebildet ist und an einem distalen Ende des Lichtleiters (30) angeordnet ist, so dass Licht aus dem Lichtleiter (30) in das optische Element einkoppelbar ist, wobei das optische Element eine Längsachse (L) definiert und eine um die Längsachse verlaufende Mantelfläche aufweist, welche zumindest abschnittsweise von mindestens einer Reflektorschicht (43) bedeckt ist, wobei das optische Element (40) einen Licht-reflektierenden Bereich aufweist, welcher von der Reflektorschicht (43) bedeckt ist, und einen Licht-durchlässigen Bereich aufweist, welcher vorzugsweise von der Reflektorschicht (43) frei bleibt, sodass das in das optische Element eingekoppelte Licht zumindest teilweise an dem Licht-reflektierenden Bereich reflektierbar ist, und Licht an dem Licht-durchlässigen Bereich abstrahlbar ist,
**dadurch gekennzeichnet, dass** die Reflektorschicht (43) eine Spiegelschicht (43.2) aufweist und zusätzlich zu der Spiegelschicht (43.2) eine unterhalb der Spiegelschicht (43.2) befindliche untere Schicht (43.1) aufweist, und wobei die Reflektivität (101) der Reflektorschicht (43) für zumindest einen Wellenlängenbereich größer als 90% ist.

2. Beleuchtungssystem (1) nach dem vorhergehenden Anspruch,
**dadurch gekennzeichnet, dass**
das Licht quer zu der Längsachse (L) des optischen Elements und seitlich über eine aktive Länge des optischen Elements abstrahlbar ist und/oder sich zumindest ein, von der Reflektorschicht (43) unbedeckter Bereich des Diffusor-Elements (40) über die Längsrichtung des Diffusor-Elements (40) erstreckt und dieser unbedeckte Bereich lichtdurchlässig ist, sodass durch das Diffusor-Element (40) geleitetes Licht streifenförmig abgebbar ist.

3. Beleuchtungssystem (1) nach einem der vorhergehenden Ansprüche,
**dadurch gekennzeichnet, dass**
die maximale Reflektivität (101) größer als 95 % und besonders bevorzugt größer als 99 % ist, insbesondere bei senkrechtem Lichteinfall auf die Reflektorschicht (43) und die Reflektivität (101) vorzugsweise gezielt auf eine definierte Wellenlänge (103) des verwendeten Lichts oder in einem vorgebbaren Bereich um eine Haupt-Wellenlänge (103) des Lichts einstellbar ist, und/oder
die Reflektivität (101) der Reflektorschicht (43) bei einem Lichteinfallswinkel von größer als 45°, bevorzugt größer als 60°, besonders bevorzugt größer als 80° bezogen auf die Senkrechte zur Reflektorschicht (43) größer ist, als 50% bevorzugt 70%, meist bevorzugt 90% der Reflektivität bei senkrechtem Lichteinfall.

4. Beleuchtungssystem (1) nach einem der vorhergehenden Ansprüche,
**dadurch gekennzeichnet, dass**
die Spiegelschicht (43.2) eine metallische Schicht aufweist oder aus dieser ausgebildet ist, welche vorzugsweise ein oder mehrere Metalle aus der Gruppe der Edelmetalle oder ein Metall der Gruppe aus Mg, Al, Cu umfasst.

5. Beleuchtungssystem (1) nach einem der vorhergehenden Ansprüche,
**dadurch gekennzeichnet, dass**
die Reflektorschicht (43) eine oberhalb der Spiegelschicht (43.2) befindliche obere Schicht (43.3) aufweist, wobei die untere Schicht (43.1) insbesondere aus einer oder mehreren Schichten besteht und wobei die obere Schicht (43.3) insbesondere aus einer oder mehreren Schichten besteht, und
wobei eine unterhalb der Spiegelschicht (43.2) befindliche untere Schicht (43.1) vorzugsweise ausgebildet ist als Haftschicht oder Haftvermittlerschicht, und/oder
wobei eine unterhalb der Spiegelschicht (43.2) befindliche untere Schicht (43.1) vorzugsweise ausgebildet ist als Schichtbereich auf dem Diffusor-Grundkörper (41) mit chemisch und/oder physikalisch veränderten Oberflächeneigenschaften, und/oder
wobei eine unterhalb der Spiegelschicht (43.2) befindliche untere Schicht (43.1) vorzugsweise ausgebildet ist als ein Grundschichtbereich bestehend aus einer einzelnen Schicht oder einer Abfolge von Schichten,
wobei der unterhalb der Spiegelschicht (43.2) befindliche Grundschichtbereich vorzugsweise eine oder mehrere auf dem optischen Element aufgebrachte Schichten umfasst und/oder eine oberflächliche Schicht des optischen Elements mit einer veränderten Oberflächeneigenschaft umfasst, welche insbesondere hergestellt oder herstellbar ist mittels eines chemischen und/oder physikalischen Prozesses zur Veränderung zumindest einer Oberflächeneigenschaft des optischen Elements.

6. Beleuchtungssystem (1) nach dem vorhergehenden Anspruch,
**dadurch gekennzeichnet, dass**
das Beleuchtungssystem (1) zumindest eines der folgenden Merkmale aufweist:
- die untere Schicht (43.1), insbesondere die Haftschicht oder Haftvermittlerschicht (43.1) oder der Grundschichtbereich, ist als dielektrische Schicht ausgebildet, wobei die dielektrische Schicht vorzugsweise Oxide, Nitride oder Oxinitride aus mindestens einem Element der Gruppe aus Si, Al, Ti, Zr, Hf, Y, Zn aufweist,
- die obere Schicht (43.2), insbesondere die Passivierungsschicht (43.3), ist als dielektrische Schicht ausgebildet, wobei die dielektrische Schicht vorzugsweise Oxide, Nitride oder Oxinitride aus mindestens einem Element der Gruppe aus Si, Al, Ti, Zr, Hf, Y, Zn aufweist.

7. Beleuchtungssystem (1) nach einem der vorhergehenden Ansprüche,
**dadurch gekennzeichnet, dass**
die Spiegelschicht (43.2) oder die Reflektorschicht (43) als ein dielektrisches Mehrschichtsystem ausgebildet ist, welches insbesondere eine Abfolge von niedrig- und hochbrechenden Metall-Oxiden und/oder -Nitriten aufweist.

8. Beleuchtungssystem (1) nach einem der vorhergehenden Ansprüche,
**dadurch gekennzeichnet, dass**
die Spiegelschicht (43.2) oder die Reflektorschicht (43) Streuzentren umfasst oder eine Schicht mit Streuzentren umfasst, wobei an den Streuzentren Licht diffus reflektierbar ist, und
wobei die Spiegelschicht (43.2) oder die Reflektorschicht (43) vorzugsweise ferner zumindest eine Schicht mit einem Metall oder einer chemischen Verbindung mit einem Metall umfasst, z.B. dielektrische Schichten umfasst, wobei an der zumindest einen Schicht mit einem Metall oder einer chemischen Verbindung mit einem Metall Licht gerichtet reflektierbar ist, und
wobei die zumindest eine Schicht mit dem Metall oder einer chemischen Verbindung mit einem Metall vorzugsweise oberhalb der Schicht mit Streuzentren angeordnet ist, insbesondere derart, dass die Schicht mit den Streuzentren außen zumindest bereichsweise umschlossen ist.

9. Beleuchtungssystem (1) nach einem der vorhergehenden Ansprüche,
**dadurch gekennzeichnet, dass**
die Schichtdicken der Schichten (43.1, 43.2, 43,3) sind nach mindestens einem der folgenden Merkmale definiert:
- die Dicke der Haftschicht (43.1) ist größer als 5 nm, bevorzugt größer als 30 nm, und/oder kleiner als 3000 nm, bevorzugt kleiner als 300 nm, bevorzugt kleiner als 150 nm,
- die Dicke der Spiegelschicht (43.2) ist größer als 10 nm, bevorzugt größer als 20 nm, bevorzugt größer als 50 nm und/oder kleiner als 5000 nm, bevorzugt kleiner als 200 nm, bevorzugt kleiner als 100 nm,
- die Dicke der Passivierungsschicht (43.3) ist größer als 5 nm, bevorzugt größer als 100 nm, bevorzugt größer als 150 nm und/oder kleiner als 5000 nm, bevorzugt kleiner als 500 nm, bevorzugt kleiner als 250 nm.

10. Beleuchtungssystem (1) nach einem der vorhergehenden Ansprüche,
**gekennzeichnet durch** zumindest eines der folgenden Merkmale:
(i) dass die Reflektorschicht (43) am distalen Ende (44) des Diffusor-Elements (40) angeordnet ist und/oder das Diffusor-Element (40) zumindest teilweise ummantelt,
(ii) dass das Diffusor-Element (40) Streuelemente aufweist, die in die Matrix des Diffusor-Elements (40) eingeschlossen sind, und/oder die Matrix des Diffusor-Elements (40) von einem Material mit Streuelementen ummantelt ist,
(iii) dass das Diffusor-Element (40) Borosilikat-, Phosphat-Kron-Glas, Blei-Silikat-Glas, Zinn-Silikat- oder Alkali-Zink-Glas und/oder die Streuelemente Weißglas-Stäbchen enthalten, die insbesondere mit einem Hüllrohr, vorzugsweise aus Borosilikatglas umschlossen sind,
(iv) dass das Diffusor-Element (40) zumindest teilweise oder abschnittsweise in seinem Volumen und/oder auf seiner Oberfläche strukturiert ist, oder das Diffusor-Element (40) eine eingefärbte oder farblose, insbesondere transparente Ummantelung, vorzugsweise aus einem eingefärbten Glas oder einem eingefärbten Kunststoff aufweist.

11. Beleuchtungssystem (1) nach mindestens einem der vorherigen Ansprüche, **dadurch gekennzeichnet, dass**
die Spiegelschicht (43.2) oder die Reflektorschicht (43) für zwei unterschiedliche Wellenlängen λ₁ und λ₂ mit einer Differenz von vorzugsweise Δλ = | λ₁ - λ₂ | < 200 nm, besonders bevorzugt Δλ = | λ₁ - λ₂ | < 100 nm, eine abweichende Reflektivität und/oder Transmission von vorzugsweise zumindest 10 %, besonders bevorzugt zumindest 30 %, aufweist und
wobei die Lichtquelle (10) des Beleuchtungssystems vorzugsweise derart eingerichtet ist, dass eine Abgabe der unterschiedlichen Wellenlängen λ₁ und λ₂ einstellbar ist, um das Beleuchtungssystem (1) in seiner Abstrahlcharakteristik umschaltbar auszubilden.

12. Verfahren zum Herstellen von einem Beleuchtungssystem (1) für ein medizintechnisches Therapie- und/oder Diagnosesystem, insbesondere zur Anwendung an lebendem Gewebe, aufweisend wenigstens eine Lichtquelle (10), einen Lichtleiter (30), der an einem proximalen Ende an die Lichtquelle (10) anschließbar oder dieser zuordenbar ist, und ein optisches Element, welches bevorzugt als Diffusor-Element (40) ausgebildet ist und an einem distalen Ende des Lichtleiters (30) angeordnet ist und Licht aus dem Lichtleiter (30) in das optische Element einkoppelbar ist, aufweisend folgende Schritte:
- Bereitstellen des optischen Elements, wobei das optische Element eine Längsachse definiert und eine um die Längsachse verlaufende Mantelfläche aufweist,
- zumindest abschnittsweise Bedecken der Mantelfläche mit einer Licht-reflektierenden Reflektorschicht (43), wobei die Reflektorschicht (43) eine Spiegelschicht (43.2) aufweist und zusätzlich zu der Spiegelschicht (43.2) eine unterhalb der Spiegelschicht (43.2) befindliche untere Schicht (43.1) aufweist, sodass das optische Element (40) einen Licht-reflektierenden Bereich aufweist, welcher von der Reflektorschicht (43) bedeckt ist, und einen Licht-durchlässigen Bereich aufweist, welcher von der Reflektorschicht (43) frei bleibt, sodass das vom optischen Element abzustrahlende Licht zumindest teilweise reflektierbar ist, und Licht gezielt am Licht-durchlässigen Bereich abstrahlbar ist, wobei die Reflektivität (101) der Reflektorschicht (43) für zumindest einen Wellenlängenbereich größer als 90% ist.

13. Verfahren nach dem vorhergehenden Anspruch,
**gekennzeichnet durch** zumindest eines der folgenden Merkmale:
(i) Vorbehandeln der Mantelfläche durch Erzeugen eines Grundschichtbereichs mit zumindest einer veränderten Oberflächeneigenschaft, insbesondere mittels chemischer oder physikalischer Prozesse zur Veränderung der Oberflächeneigenschaften der Mantelfläche,
(ii) Aufbringen einer Reflektorschicht (43) auf der Mantelfläche, insbesondere mittels Aufdampfen, Kathoden-Zerstäubung, Hochfrequenz-Zerstäubung, Reaktiv-Zerstäubung und/oder Magnetron-Zerstäubung,
(iii) Aufbringen einer Haftschicht (43.1) oder einer Haftvermittlerschicht auf der Mantelfläche und/oder Aufbringen einer Passivierungsschicht (43.3) auf der Reflektorschicht (43), vorzugsweise mittels Aufdampfen, Kathoden-Zerstäubung, Hochfrequenz-Zerstäubung, Reaktiv-Zerstäubung und/oder Magnetron-Zerstäubung.

14. Verfahren nach dem vorhergehenden Anspruch umfassend:
Verbinden des optischen Elements mit dem distalen Ende des Lichtleiters (30) indem in einem ersten Verbindungsschritt ein erstes Lichtleiter-Teilstück mit dem optischen Element verbunden wird und danach in einem zweiten Verbindungsschritt ein zweites, insbesondere längeres, Lichtleiter-Teilstück mit dem ersten Lichtleiter-Teilstück verbunden wird und
wobei vorzugsweise das zumindest abschnittsweise Bedecken der Mantelfläche mit einer Licht-reflektierenden Reflektorschicht zwischen dem ersten Verbindungsschritt und dem zweiten Verbindungsschritt erfolgt.

15. Vorrichtung umfassend ein Beleuchtungssystems (1) nach einem der Ansprüche 1 bis 11 als Komponente wobei die Vorrichtung ausgebildet ist als:
(i) Vorrichtung für ein medizinisches Therapieverfahren, insbesondere für eine photodynamische Therapie (PDT) oder photo-Immuno Therapie (PIT) zur Tumortherapie, für eine endovenöse Lasertherapie (EVLT) zur Behandlung von Krampfadern, für eine Laser- induzierte interstitielle Thermotherapie (LITT) oder für Anwendungen im Bereich der Dentalmedizin, Augenheilkunde sowie Dermatologie,
(ii) Vorrichtung für eine photodynamische Therapie (PDT) zur Tumortherapie, wobei zumindest ein Lichtleiter (30) mit dem Diffusor-Element (40) aus anderen Diffusor-Elementen (40) abgestrahltes Licht aufnimmt und über den Lichtleiter (30) einem Detektor zur spektroskopischen Analyse und/ oder für eine dosimetrische Messung weiterleitet,
(iii) Vorrichtung für industrielle Anwendungen zur gezielten Ausleuchtung von Hohlräumen oder zur Bestrahlung von Proben im Rahmen der In-Vitro-Diagnostik.

## Claims

1. A lighting system (1), in particular for a medical technology therapy and/or diagnosis system, comprising at least one light source (10), a light guide (30) which is connectable to the light source (10) at a proximal end thereof or assignable thereto, and an optical element, preferably in the form of a diffuser element (40) and arranged at a distal end of the light guide (30), so that light from the light guide (30) can be injected into the optical element, said optical element defining a longitudinal axis (L) and having a lateral surface surrounding the longitudinal axis and being covered by at least one reflector layer (43) at least in sections thereof, wherein the optical element (40) comprises a light-reflecting area covered by the reflector layer (43), and comprises a light-transmissive area which preferably remains free of the reflector layer (43), so that the light injected into the optical element can be reflected at least partially at the light-reflecting area and light can be emitted from the light-transmissive area;
**characterised in that** the reflector layer (43) comprises a mirror layer (43.2) and, in addition to the mirror layer (43.2), comprises a lower layer (43.1) disposed below the mirror layer (43.2), and wherein the reflector layer (43) exhibits a reflectance (101) of more than 90 % for at least one wavelength range.

2. The lighting system (1) according to the preceding claim,
**characterised in that**
the light can be emitted transversely to the longitudinal axis (L) of the optical element and laterally over an active length of the optical element; and/or
at least one area of the diffuser element (40) that is not covered by the reflector layer (43) extends along the longitudinal extension of the diffuser element (40) and this non-covered area is light-transmissive, so that light carried through the diffuser element (40) can be emitted in a strip-like pattern.

3. The lighting system (1) according to any one of the preceding claims,
**characterised in that**
maximum reflectance (101) is greater than 95 % and most preferably greater than 99 %, in particular for perpendicular incidence of light onto the reflector layer (43), and that the reflectance (101) is preferably selectively adjustable to a predefined wavelength (103) of the employed light or in a predetermined range around a main wavelength (103) of the light; and/or
for a light incidence angle of greater than 45°, preferably greater than 60°, most preferably greater than 80° with respect to the perpendicular to the reflector layer (43), the reflector layer (43) exhibits a reflectance (101) of more than 50 %, preferably 70 %, most preferably 90 % of the reflectance at perpendicular incidence of light.

4. The lighting system (1) according to any one of the preceding claims,
**characterised in that**
the mirror layer (43.2) comprises or is made of a metallic layer which preferably contains one or more metals from the group of noble metals or a metal from the group consisting of Mg, Al, Cu.

5. The lighting system (1) according to any one of the preceding claims,
**characterised in that**
the reflector layer (43) comprises an upper layer (43.3) provided above the mirror layer (43.2), wherein said lower layer (43.1) in particular consists of one or more layers and wherein said upper layer (43.3) in particular consists of one or more layers; and
wherein a lower layer (43.1) provided below the mirror layer (43.2) is preferably in the form of an adhesion layer or adhesion promoting layer; and/or
wherein a lower layer (43.1) provided below the mirror layer (43.2) is preferably in the form of a layer zone on the diffuser base body (41) and has chemically and/or physically modified surface properties; and/or
wherein a lower layer (43.1) provided below the mirror layer (43.2) is preferably in the form of a base layer zone consisting of a single layer or of a sequence of layers;
wherein said base layer zone provided below the mirror layer (43.2) preferably comprises one or more layers applied on the optical element and/or comprises a near-surface layer of the optical element, which has a modified surface property, which in particular is produced or can be produced by a chemical and/or physical process for altering at least one surface property of the optical element.

6. The lighting system (1) according to the preceding claim,
**characterised in that**
the lighting system (1) exhibits at least one of the following features:
- the lower layer (43.1), in particular the adhesion layer or adhesion promoting layer (43.1) or the base layer zone, is in the form of a dielectric layer, said dielectric layer preferably including oxides, nitrides, or oxynitrides of at least one element from the group consisting of Si, Al, Ti, Zr, Hf, Y, Zn;
- the upper layer (43.3), in particular the passivation layer (43.3), is in the form of a dielectric layer, said dielectric layer preferably including oxides, nitrides, or oxynitrides of at least one element from the group consisting of Si, Al, Ti, Zr, Hf, Y, Zn.

7. The lighting system (1) according to any one of the preceding claims,
**characterised in that**
the mirror layer (43.2) or the reflector layer (43) is in the form of a dielectric multilayer system comprising in particular a sequence of low refractive index and high refractive index metal oxides and/or metal nitrites.

8. The lighting system (1) according to any one of the preceding claims,
**characterised in that**
the mirror layer (43.2) or the reflector layer (43) includes scattering centres or comprises a layer including scattering centres, said scattering centres allowing light to be reflected diffusely thereon; and
wherein the mirror layer (43.2) or the reflector layer (43) preferably furthermore comprise at least one layer including a metal or including a chemical compound comprising a metal, for example comprising dielectric layers, wherein said at least one layer including a metal or including a chemical compound comprising a metal allows light to be reflected thereon in a directed manner; and
wherein the at least one layer including the metal or including a chemical compound comprising a metal is preferably disposed above the layer that includes scattering centres, in particular in such a way that the layer including the scattering centres is at least partially enclosed on the outside.

9. The lighting system (1) according to any one of the preceding claims,
**characterised in that**
the layer thicknesses of the layers (43.1, 43.2, 43.3) are defined according to at least one of the following features:
- the adhesion layer (43.1) has a thickness of more than 5 nm, preferably more than 30 nm, and/or of less than 3000 nm, preferably less than 300 nm, preferably less than 150 nm;
- the mirror layer (43.2) has a thickness of more than 10 nm, preferably more than 20 nm, preferably more than 50 nm, and/or of less than 5000 nm, preferably less than 200 nm, preferably less than 100 nm;
- the passivation layer (43.3) has a thickness of more than 5 nm, preferably more than 100 nm, preferably more than 150 nm, and/or of less than 5000 nm, preferably less than 500 nm, preferably less than 250 nm.

10. The lighting system (1) according to any one of the preceding claims,
**characterised by** at least one of the following features:
(i) the reflector layer (43) is arranged at the distal end (44) of the diffuser element (40) and/or at least partially encloses the diffuser element (40) laterally;
(ii) the diffuser element (40) comprises scattering elements which are included in the matrix of the diffuser element (40), and/or the matrix of the diffuser element (40) is laterally enclosed by a material that includes scattering elements;
(iii) the diffuser element (40) comprises borosilicate glass or phosphate crown glass or lead silicate glass or tin silicate glass or alkali zinc glass, and/or the scattering elements comprise white glass rods, in particular enclosed by a cladding tube preferably made of borosilicate glass;
(iv) the diffuser element (40) is structured at least partially or in sections of its volume and/or on its surface, or the diffuser element (40) comprises a coloured or colourless, in particular transparent jacket, preferably made of a coloured glass or a coloured plastics material.

11. The lighting system (1) according to at least one of the preceding claims,
**characterised in that**
the mirror layer (43.2) or the reflector layer (43) exhibit a reflectance and/or transmittance that is different by preferably at least 10 %, most preferably by at least 30 %, for two different wavelengths λ₁ and λ₂ with a difference of preferably Δλ = |λ₁ - λ₂| < 200 nm, most preferably Δλ = |λ₁ - λ₂| < 100 nm; and
wherein the light source (10) of the lighting system is preferably adapted so that an emission of the different wavelengths λ₁ and λ₂ can be set, so that the lighting system (1) can switch between different emission characteristics.

12. A method for producing a lighting system (1) for a medical technology therapy and/or diagnosis system, in particular for use on living tissue, comprising at least one light source (10), a light guide (30) which is connectable to the light source (10) at a proximal end thereof or assignable thereto, and an optical element, preferably in the form of a diffuser element (40) and arranged at a distal end of the light guide (30), and wherein light from the light guide (30) can be injected into the optical element, comprising the steps of:
- providing the optical element, said optical element defining a longitudinal axis and having a lateral surface surrounding the longitudinal axis;
- covering the lateral surface at least partially with a light-reflecting reflector layer (43), wherein said reflector layer (43) comprises a mirror layer (43.2) and, in addition to the mirror layer (43.2), comprises a lower layer (43.1) disposed below the mirror layer (43.2), so that the optical element (40) comprises a light-reflecting area that is covered by the reflector layer (43), and comprises a light-transmissive area which remains free of the reflector layer (43), so that the light to be emitted by the optical element can be reflected at least partially and light can be selectively emitted from the light-transmissive area, wherein the reflector layer (43) exhibits a reflectance (101) of more than 90 % for at least one wavelength range.

13. The method according to the preceding claim,
**characterised by** at least one of the following features:
(i) pre-treating the lateral surface by producing a base layer zone having at least one modified surface property, in particular by chemical or physical processes, for modifying the surface properties of the lateral surface;
(ii) applying a reflector layer (43) onto the lateral surface, in particular by vapour deposition, cathode sputter deposition, high-frequency sputter deposition, reactive sputter deposition and/or magnetron sputter deposition;
(iii) applying an adhesion layer (43.1) or an adhesion promoting layer onto the lateral surface and/or applying a passivation layer (43.3) onto the reflector layer (43), preferably by vapour deposition, cathode sputter deposition, high-frequency sputter deposition, reactive sputter deposition and/or magnetron sputter deposition.

14. The method according to the preceding claim, comprising:
connecting the optical element to the distal end of the light guide (30) by connecting a first light guide portion to the optical element in a first connecting step and then connecting a second, in particular longer light guide portion to the first light guide portion in a second connecting step; and
wherein, preferably, the covering of the lateral surface at least partially with a light-reflecting reflector layer is effected between the first connecting step and the second connecting step.

15. A device, comprising a lighting system (1) according to any one of claims 1 to 11 as a component, the device being in the form of:
(i) a device for a medical therapy method, in particular for photodynamic therapy (PDT) or photoimmunotherapy (PIT) for tumour treatment, for endovenous laser therapy (EVLT) for the treatment of varicose veins, for laser-induced interstitial thermal therapy (LITT) or for applications in the field of dental medicine, ophthalmology, and dermatology;
(ii) a device for photodynamic therapy (PDT) for tumour treatment, wherein at least one light guide (30) with the diffuser element (40) captures light emitted from other diffuser elements (40) to forward it via the light guide (30) to a detector for spectroscopic analysis and/or for a dosimetric measurement;
(iii) a device for industrial applications for selectively illuminating cavities or for irradiation of samples in the context of in-vitro diagnostics.

## Revendications

1. Système d'éclairage (1), en particulier pour un système de thérapie et/ou de diagnostic en technologie médicale, présentant au moins une source de lumière (10), un guide de lumière (30) qui peut être raccordé ou associé à la source de lumière (10) à une extrémité proximale, et un élément optique, lequel est de préférence réalisé en tant qu'élément diffuseur (40) et est disposé à une extrémité distale du guide de lumière (30), de sorte que de la lumière provenant du guide de lumière (30) puisse être injectée dans l'élément optique, dans lequel l'élément optique définit un axe longitudinal (L) et présente une surface d'enveloppe s'étendant autour de l'axe longitudinal, laquelle est recouverte au moins sur certaines sections par au moins une couche réflectrice (43), dans lequel l'élément optique (40) présente une zone réfléchissant la lumière, laquelle est recouverte par la couche réflectrice (43), et présente une zone translucide, laquelle reste de préférence dégagée de la couche réflectrice (43), de sorte que la lumière injectée dans l'élément optique puisse être au moins partiellement réfléchie sur la zone réfléchissant la lumière, et que la lumière puisse être émise sur la zone translucide,
**caractérisé en ce que** la couche réflectrice (43) présente une couche réfléchissante (43.2) et en plus de la couche réfléchissante (43.2) une couche inférieure (43.1) située au-dessous de la couche réfléchissante (43.2), et dans lequel la réflectivité (101) de la couche réflectrice (43) est supérieure à 90 % pour au moins une plage de longueurs d'onde.

2. Système d'éclairage (1) selon la revendication précédente,
**caractérisé en ce que**
la lumière peut être émise transversalement à l'axe longitudinal (L) de l'élément optique et latéralement sur une longueur active de l'élément optique et/ou au moins une zone de l'élément diffuseur (40) non recouverte par la couche réflectrice (43) s'étend sur la direction longitudinale de l'élément diffuseur (40) et cette zone non recouverte est translucide, de sorte que la lumière dirigée par l'élément diffuseur (40) puisse être délivrée sous forme de bandes.

3. Système d'éclairage (1) selon l'une quelconque des revendications précédentes,
**caractérisé en ce que**
la réflectivité maximale (101) est supérieure à 95 % et de manière particulièrement préférée supérieure à 99 %, en particulier en cas d'incidence verticale de la lumière sur la couche réflectrice (43), et la réflectivité (101) peut être réglée de préférence de manière ciblée sur une longueur d'onde (103) définie de la lumière utilisée ou dans une plage pouvant être prédéfinie autour d'une longueur d'onde principale (103) de la lumière, et/ou
la réflectivité (101) de la couche réflectrice (43) est supérieure à 50 %, de préférence à 70 %, et généralement de préférence à 90 % de la réflectivité en cas d'incidence verticale de la lumière, lorsque l'angle d'incidence de la lumière est supérieur à 45°, de préférence supérieur à 60°, et de manière particulièrement préférée supérieur à 80° par rapport à la perpendiculaire à la couche réflectrice (43) .

4. Système d'éclairage (1) selon l'une quelconque des revendications précédentes,
**caractérisé en ce que**
la couche réfléchissante (43.2) présente une couche métallique ou est réalisée à partir de celle-ci, laquelle comprend de préférence un ou plusieurs métaux du groupe des métaux précieux ou un métal du groupe des Mg, Al, Cu.

5. Système d'éclairage (1) selon l'une quelconque des revendications précédentes,
**caractérisé en ce que**
la couche réflectrice (43) présente une couche supérieure (43.3) située au-dessus de la couche réfléchissante (43.2), dans lequel la couche inférieure (43.1) est en particulier constituée d'une ou plusieurs couches et dans lequel la couche supérieure (43.3) est en particulier constituée d'une ou plusieurs couches, et
dans lequel une couche inférieure (43.1) située au-dessous de la couche réfléchissante (43.2) est de préférence réalisée en tant que couche adhésive ou couche d'agent adhésif, et/ou
dans lequel une couche inférieure (43.1) située au-dessous de la couche réfléchissante (43.2) est de préférence réalisée en tant que zone de couche sur le corps de base de diffuseur (41) avec des propriétés de surface chimiquement et/ou physiquement modifiées, et/ou
dans lequel une couche inférieure (43.1) située au-dessous de la couche réfléchissante (43.2) est de préférence réalisée comme une zone de couche de base constituée d'une seule couche ou d'une succession de couches,
dans lequel la zone de couche de base située au-dessous de la couche réfléchissante (43.2) comprend de préférence une ou plusieurs couches appliquées sur l'élément optique et/ou comprend une couche superficielle de l'élément optique avec une propriété de surface modifiée, laquelle en particulier est fabriquée ou peut être fabriquée au moyen d'un processus chimique et/ou physique visant à modifier au moins une propriété de surface de l'élément optique.

6. Système d'éclairage (1) selon la revendication précédente,
**caractérisé en ce que**
le système d'éclairage (1) présente au moins l'une des caractéristiques suivantes :
- la couche inférieure (43.1), en particulier la couche adhésive ou couche d'agent adhésif (43.1) ou la zone de couche de base, est réalisée en tant que couche diélectrique, dans lequel la couche diélectrique présente de préférence des oxydes, des nitrures ou des oxynitrures composés d'au moins un élément du groupe des Si, Al, Ti, Zr, Hf, Y, Zn,
- la couche supérieure (43.2), en particulier la couche de passivation (43.3), est réalisée en tant que couche diélectrique, dans lequel la couche diélectrique présente de préférence des oxydes, des nitrures ou des oxynitrures composés d'au moins un élément du groupe des Si, Al, Ti, Zr, Hf, Y, Zn.

7. Système d'éclairage (1) selon l'une quelconque des revendications précédentes,
**caractérisé en ce que**
la couche réfléchissante (43.2) ou la couche réflectrice (43) est réalisée comme un système multicouche diélectrique, lequel présente en particulier une succession d'oxydes et/ou de nitrites métalliques à faible et à fort indice de réfraction.

8. Système d'éclairage (1) selon l'une quelconque des revendications précédentes,
**caractérisé en ce que**
la couche réfléchissante (43.2) ou la couche réflectrice (43) comprend des centres de dispersion ou comprend une couche avec des centres de dispersion, dans lequel de la lumière peut être réfléchie de manière diffuse sur les centres de dispersion, et
dans lequel la couche réfléchissante (43.2) ou la couche réflectrice (43) comprend de préférence en outre au moins une couche avec un métal ou un composé chimique avec un métal, par exemple des couches diélectriques, dans lequel de la lumière peut être réfléchie de manière dirigée sur l'au moins une couche avec un métal ou un composé chimique avec un métal, et
dans lequel l'au moins une couche avec le métal ou un composé chimique avec un métal est de préférence disposée au-dessus de la couche avec les centres de dispersion, en particulier de telle sorte que la couche avec les centres de dispersion soit extérieurement entourée au moins par endroits.

9. Système d'éclairage (1) selon l'une quelconque des revendications précédentes,
**caractérisé en ce que**
les épaisseurs de couche des couches (43.1, 43.2, 43,3) sont définies selon au moins l'une des caractéristiques suivantes :
- l'épaisseur de la couche adhésive (43.1) est supérieure à 5 nm, de préférence supérieure à 30 nm, et/ou inférieure à 3000 nm, de préférence inférieure à 300 nm, de préférence inférieure à 150 nm,
- l'épaisseur de la couche réfléchissante (43.2) est supérieure à 10 nm, de préférence supérieure à 20 nm, de préférence supérieure à 50 nm et/ou inférieure à 5000 nm, de préférence inférieure à 200 nm, de préférence inférieure à 100 nm,
- l'épaisseur de la couche de passivation (43.3) est supérieure à 5 nm, de préférence supérieure à 100 nm, de préférence supérieure à 150 nm et/ou inférieure à 5000 nm, de préférence inférieure à 500 nm, de préférence inférieure à 250 nm.

10. Système d'éclairage (1) selon l'une quelconque des revendications précédentes, **caractérisé par** au moins l'une des caractéristiques suivantes :
(i) que la couche réflectrice (43) est disposée à l'extrémité distale (44) de l'élément diffuseur (40) et/ou enveloppe au moins partiellement l'élément diffuseur (40),
(ii) que l'élément diffuseur (40) présente des éléments de dispersion qui sont inclus dans la matrice de l'élément diffuseur (40) et/ou la matrice de l'élément diffuseur (40) est enveloppée d'un matériau avec des éléments de dispersion,
(iii) que l'élément diffuseur (40) contient du verre crown borosilicaté, du verre crown phosphaté, du verre de silicate de plomb, du verre de silicate d'étain ou alcali-zinc et/ou les éléments de dispersion contiennent des bâtonnets de verre blanc qui sont entourés en particulier d'un tube d'enveloppe, de préférence en verre borosilicaté,
(iv) que l'élément diffuseur (40) est structuré au moins partiellement ou sur certaines sections dans son volume et/ou sur sa surface, ou l'élément diffuseur (40) présente une enveloppe colorée ou incolore, en particulier transparente, de préférence composée d'un verre coloré ou d'un plastique coloré.

11. Système d'éclairage (1) selon au moins l'une des revendications précédentes, **caractérisé en ce que**
la couche réfléchissante (43.2) ou la couche réflectrice (43) pour deux longueurs d'onde différentes λ₁ et λ₂ avec une différence de préférence de Δλ = |λ₁ - λ₂| < 200 nm, de manière particulièrement préférée de Δλ = |λ₁ - λ₂| < 100 nm, présente une réflectivité et/ou transmission divergente de préférence d'au moins 10 %, de manière particulièrement préférée d'au moins 30 % et
dans lequel la source de lumière (10) du système d'éclairage est de préférence conçue de telle sorte qu'une délivrance des différentes longueurs d'onde λ₁ et λ₂ est réglable, afin de réaliser le système d'éclairage (1) commutable dans sa caractéristique d'émission.

12. Procédé de fabrication d'un système d'éclairage (1) pour un système de thérapie et/ou de diagnostic en technologie médicale, en particulier pour une application sur des tissus vivants, présentant au moins une source de lumière (10), un guide de lumière (30) qui peut être raccordé ou peut être associé à la source de lumière (10) à une extrémité proximale, et un élément optique, lequel de préférence est réalisé en tant qu'élément diffuseur (40) et est disposé à une extrémité distale du guide de lumière (30) et de la lumière peut être injectée à partir du guide de lumière (30) dans l'élément optique, présentant les étapes suivantes :
- la fourniture de l'élément optique, dans lequel l'élément optique définit un axe longitudinal et présente une surface d'enveloppe s'étendant autour de l'axe longitudinal,
- le recouvrement au moins sur certaines sections de la surface d'enveloppe avec une couche réflectrice (43) réfléchissant la lumière, dans lequel la couche réflectrice (43) présente une couche réfléchissante (43.2) et en plus de la couche réfléchissante (43.2) une couche inférieure (43.1) située au-dessous de la couche réfléchissante (43.2), de sorte que l'élément optique (40) présente une zone réfléchissant la lumière, laquelle est recouverte par la couche réflectrice (43), et présente une zone translucide, laquelle reste dégagée de la couche réflectrice (43), de sorte que la lumière devant être émise par l'élément optique puisse être au moins partiellement réfléchie, et que la lumière puisse être émise de manière ciblée sur la zone translucide, dans lequel la réflectivité (101) de la couche réflectrice (43) est supérieure à 90 % pour au moins une plage de longueurs d'onde.

13. Procédé selon la revendication précédente
**caractérisé par** au moins l'une des caractéristiques suivantes :
(i) le prétraitement de la surface d'enveloppe par production d'une zone de couche de base avec au moins une propriété de surface modifiée, en particulier au moyen de processus chimiques ou physiques visant à modifier les propriétés de surface de la surface d'enveloppe,
(ii) l'application d'une couche réflectrice (43) sur la surface d'enveloppe, en particulier par métallisation sous vide, pulvérisation cathodique, pulvérisation haute fréquence, pulvérisation réactive et/ou pulvérisation magnétron,
(iii) l'application d'une couche adhésive (43.1) ou d'une couche d'agent adhésif sur la surface d'enveloppe et/ou application d'une couche de passivation (43.3) sur la couche réflectrice (43), de préférence par métallisation sous vide, pulvérisation cathodique, pulvérisation haute fréquence, pulvérisation réactive et/ou pulvérisation magnétron.

14. Procédé selon la revendication précédente comprenant :
la liaison de l'élément optique à l'extrémité distale du guide de lumière (30) en reliant, dans une première étape de liaison, une première partie de guide de lumière à l'élément optique et en reliant ensuite, dans une deuxième étape de liaison, une deuxième partie de guide de lumière, en particulier plus longue, à la première partie de guide de lumière et
dans lequel de préférence le recouvrement au moins sur certaines sections de la surface d'enveloppe avec une couche réflectrice réfléchissant la lumière s'effectue entre la première étape de liaison et la deuxième étape de liaison.

15. Dispositif comprenant un système d'éclairage (1) selon l'une quelconque des revendications 1 à 11 en tant que composant dans lequel le dispositif est réalisé en tant que :
(i) dispositif pour une procédure thérapeutique médicale, en particulier pour une thérapie photodynamique (PDT) ou photoimmunothérapie (PIT) pour le traitement de tumeurs, pour une thérapie laser endoveineuse (EVLT) pour le traitement de varices, pour une thermothérapie interstitielle induite par laser (LITT) ou pour des applications dans le domaine de la médecine dentaire, de l'ophtalmologie ainsi que de la dermatologie,
(ii) dispositif pour une thérapie photodynamique (PDT) pour le traitement de tumeurs, dans lequel au moins un guide de lumière (30) avec l'élément diffuseur (40) reçoit de la lumière émise à partir d'autres éléments diffuseurs (40) et la transmet via le guide de lumière (30) à un détecteur pour une analyse spectroscopique et/ou pour une mesure dosimétrique,
(iii) dispositif pour des applications industrielles visant à l'éclairage ciblé de cavités ou à l'irradiation d'échantillons dans le cadre du diagnostic in vitro.
